# EUROPEAN PATENT SPECIFICATION

(11) **EP 1 861 100 B1**
(45) Date of publication and mention of the grant of the patent: **29.10.2008**
(21) Application number: 06765213.1
(22) Date of filing: 04.08.2006
(51) Int. Cl.: A61K 31/473, A61P 19/02, A61P 29/00, A61P 35/00

(54) **3,11B-CIS-DIHYDROTETRABENAZINE FOR THE TREATMENT OF A PROLIFERATIVE DISEASE OR AN INFLAMMATION**
3,11B-CIS-DIHYDROTETRABENAZIN ZUR BEHANDLUNG VON PROLIFERATIVEN KRANKHEITEN ODER ENTZÜNDUNGEN
3,11 B-CIS-DIHYDROTETRABENAZINE POUR LE TRAITEMENT D'UNE MALADIE PROLIFERATIVE OU D'UNE INFLAMMATION

(30) Priority: 05.08.2005 GB 0516168
(43) Date of publication of application: 05.12.2007
(60) Divisional application: 08104275.6
(73) Proprietor: Cambridge Laboratories (Ireland) Limited, Dublin 4 (IE)
(72) Inventor: DUFFIELD, Andrew, John, Tyne and Wear NE28 9NX (GB); YARROW, Jean Elisabeth, 24 Blendheim Gardens, Brixton SW2 5BZ (GB)
(74) Representative: Hutchins, Michael Richard
(86) International application number: PCT/GB2006/002909
(87) International publication number: WO 2007/017643

(56) References cited:
- WO-A-20/05077946
- MEHVAR R ET AL: "CONCENTRATION-EFFECT RELATIONSHIPS OF TETRABENAZINE AND DIHYDROTETRABENAZINE IN THE RAT" JOURNAL OF PHARMACEUTICAL SCIENCES, vol. 76, no. 6, 1987, pages 461-465, XP009075267 ISSN: 0022-3549 cited in the application
- SCHAEFER A ET AL: "Effects of biogenic amines and psychotropic drugs on endogenous prostaglandin biosynthesis in the rat brain homogenates" BIOCHEMICAL PHARMACOLOGY 1978 UNITED KINGDOM, vol. 27, no. 2, 1978, pages 213-218, XP009075242

## Description

This invention relates to the use of a dihydrotetrabenazine in the prophylaxis or treatment of inflammatory diseases.

### Background of the Invention

Sigma receptors are cell surface receptors that were once considered to be a subtype of opiate receptors but it has now been demonstrated, following characterization of the receptor and the discovery of specific ligands for the receptors, that they are distinct from opiate receptors (see Bourrie et al., Current Opinion in Investigational Drugs, 5(11):1158-63) (2004)).

Sigma receptors can be classified into the sigma-1 (σ-1) and sigma-2 (σ-2) subtypes. The σ-1 receptor, which is believed to contain 223 amino acids, has been cloned (Hanner et al., Proc. Natl. Acad Sci. USA, (1996) 93:8072-8077) and it has been found to exhibit no primary sequence homology to any other receptor class. However, it does have a 30.3% identity to the sequence of a fungal sterol C8-C7 isomerase and has also been found to be related to another protein of unknown function, SRBP2 (SR-31747 binding-protein 2), which shares a high homology with this enzyme. The σ-2 receptor has not yet been cloned.

Sigma receptor ligands have been reported as having anti-inflammatory activity.
For example, Bourrie et al., Eur. J. Pharmacol, (2002), 456(1-3):123-31 report that the Sanofi-Synthelabo Recherche compound SSR125329A (chemical name [(Z)-3-(4-adamantan-2-yl-3,5-dichloro-phenyl)-allyl]-cyclohexyl-ethylamine) is a high affinity sigma receptor ligand with potent anti-inflammatory properties. Bourrie *et al.*, conclude that the results of their studies provide substantial evidence that sigma receptor ligands may represent a new effective approach for rheumatoid arthritis treatment.

Another Sanofi sigma receptor antagonist (SR31747) is currently in clinical trials for the treatment of rheumatoid arthritis.

Tetrabenazine (Chemical name: 1, 3, 4,6,7,11b-hexahydro-9,10-dimethoxy-3-(2-methylpropyl)-2H-benzo(a)quinolizin-2-one) has been in use as a pharmaceutical drug since the late 1950s. Initially developed as an anti-psychotic,tetrabenazine is currently used in the symptomatic treatment of hyperkinetic movement disorders such as Huntington's disease, hemiballismus, senile chorea, tic, tardive dyskinesia and Tourette's syndrome, see for example Jankovic et al., Am. J. Psychiatry. (1999) Aug; 156(8):1279-81 and Jankovic et al., Neurology (1997) Feb; 48(2):358-62.

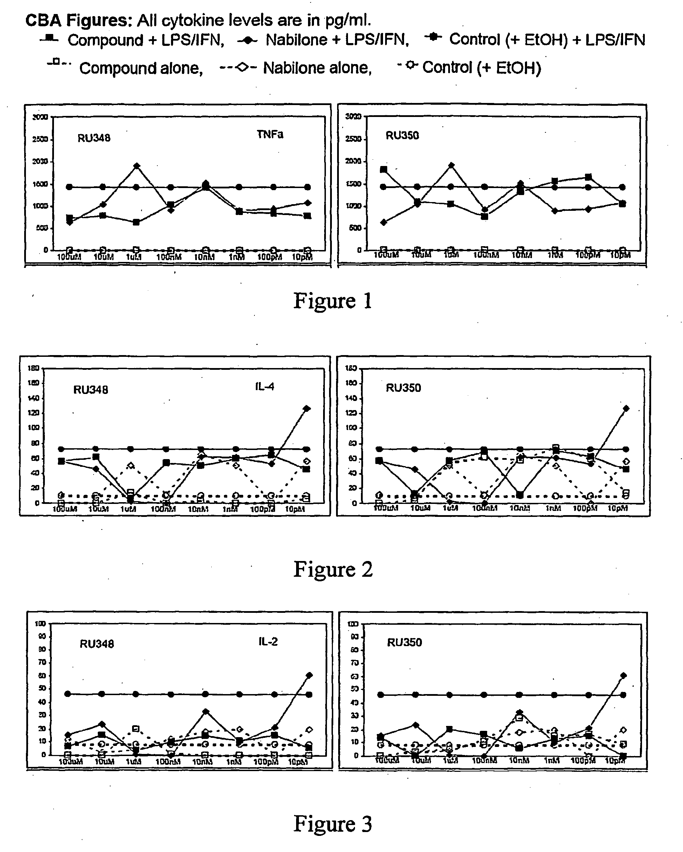

The chemical structure of tetrabenazine is as shown in Figure 1 below.

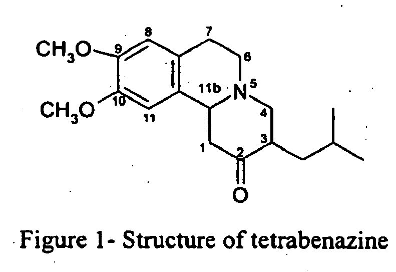

The compound has chiral centres at the 3 and 11b carbon atoms and hence can, theoretically, exist in a total of four isomeric forms, as shown in Figure 2.

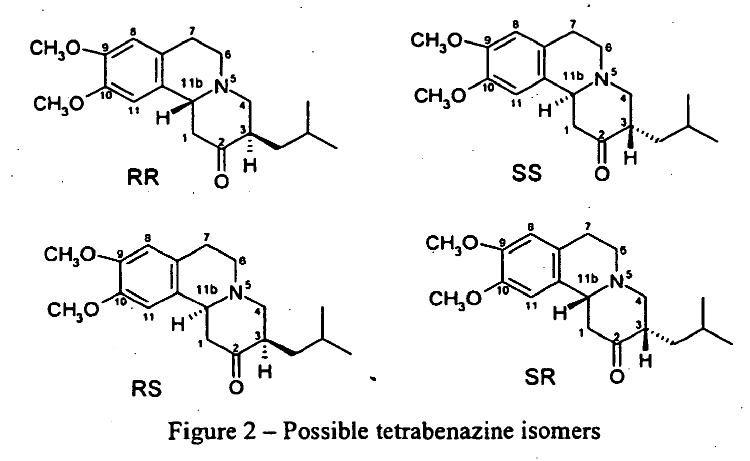

In Figure 2, the stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In Figure 2 and elsewhere in this patent application, the designations "R" or "S" are given in the order of the position numbers of the carbon atoms. Thus, for example, *RS* is a shorthand notation for 3*R*,11b*S*. Similarly, when three chiral centres are present, as in the dihydrotetrabenazines described below, the designations "*R*" or "*S*" are listed in the order of the carbon atoms 2, 3 and 11b. Thus, the 2*S*,3*R,11bR* isomer is referred to in short hand form as *SRR* and so on.

Commercially available tetrabenazine is a racemic mixture of the *RR* and *SS* isomers and it would appear that the *RR* and *SS* isomers (hereinafter referred to individually or collectively as *trans*-tetrabenazine because the hydrogen atoms at the 3 and 11b positions have a *trans* relative orientation) are the most thermodynamically stable isomers.

Tetrabenazine has somewhat poor and variable bioavailability. It is extensively metabolised by first-pass metabolism, and little or no unchanged tetrabenazine is typically detected in the urine. The major metabolite is dihydrotetrabenazine (Chemical name: 2-hydroxy-3-(2-methylpropyl)-1,3,4,6,7,11b-hexahydro-9,10-dimethoxy-benzo(a)quinolizine) which is formed by reduction of the 2-keto group in tetrabenazine, and is believed to be primarily responsible for the activity of the drug (see Mehvar et al., Drug Metab.Disp, 15, 250-255 (1987) and J. Pharm. Sci., 76, No.6, 461-465 (1987)).

Four dihydrotetrabenazine isomers have previously been identified and characterised, that are derived from the more stable *RR* and *SS* isomers of the parent tetrabenazine and have a *trans* relative orientation between the hydrogen atoms at the 3 and 11b positions) (see Kilbourn et al., Chirality, 9:59-62 (1997) and Brossi et al., Helv. Chim. Acta., vol. XLI, No. 193, pp1793-1806 (1958). The four isomers are (+)-α-dihydrotetrabenazine, (-)-α-dihydrotetrabenazine, (+)-β-dihydrotetrabenazine and (-)-β-dihydrotetrabenazine. The structures of the four known *trans*-dihydrotetrabenazine isomers are considered to be as shown in Figure 3.

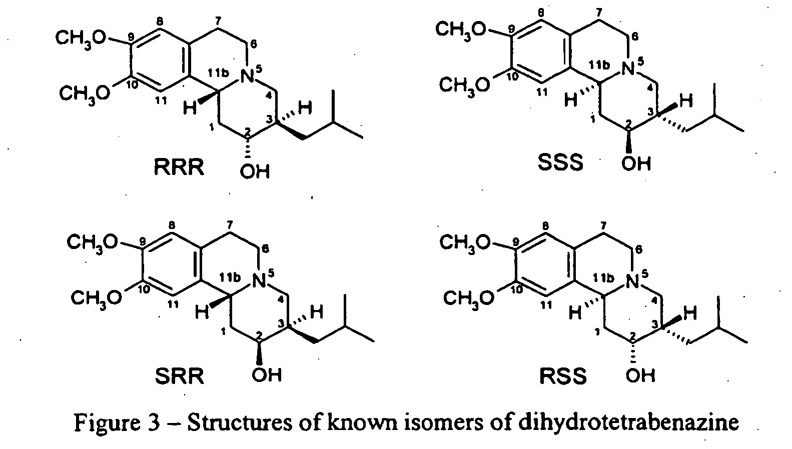

Kilbourn *et* al.,(see Eur. J. Pharmacol., 278:249-252 (1995) and Med Chem. Res., 5:113-126 (1994)) investigated the specific binding of individual radio-labelled dihydrotetrabenazine isomers in the conscious rat brain. They found that the (+)-α-[¹¹C]dihydrotetrabenazine (2*R*,3*R,*11b*R*) isomer accumulated in regions of the brain associated with higher concentrations of the neuronal membrane dopamine transporter (DAT) and the vesicular monoamine transporter (VMAT2). However, the essentially inactive (-)-α-[¹¹C]dihydrotetrabenazine isomer was almost uniformly distributed in the brain, suggesting that specific binding to DAT and VMAT2 was not occurring. The *in vivo* studies correlated with *in vitro* studies which demonstrated that the (+)-α-[¹¹C]dihydrotetrabenazine isomer exhibits a Kᵢ for [³H]methoxytetrabenazine >2000-fold higher than the Kᵢ for the (-)-α-[¹¹C]dihydrotetrabenazine isomer.

Our earlier International patent application No. PCT/GB2005/000464 discloses the preparation and therapeutic uses of dihydrotetrabenazine isomers (3,11b*-cis-*dihydrotetrabenazine) in which the hydrogen atoms at the 3 and 11b positions have a *cis* relative orientation.

There are four possible isomers of dihydrotetrabenazine having the 3,11b*-cis* configuration and these are the 2*S*,3*S*,11b*R* isomer, the 2*R*,3*R*,11b*S* isomer, the 2*R*,3*S*,11b*R* isomer and the 2*S*,3*R*,11b*S* isomer which have the following structures:
(a) the 2*S*,3*S*,11b*R* isomer of 3,11b-*cis*-dihydrotetrabenazine having the formula (Ia): 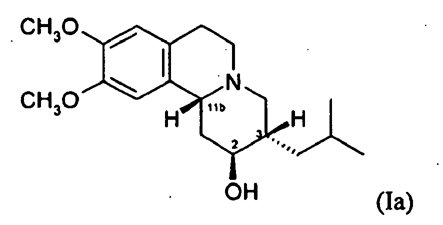
(b) the 2*R*,3*R*,11b*S* isomer of 3,11b-*cis*-dihydrotetrabenazine having the formula (Ib): 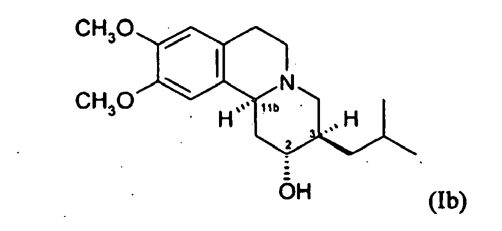
(c) the 2*R*,3*S*,11b*R* isomer of 3,11b-*cis*-dihydrotetrabenazine having the formula 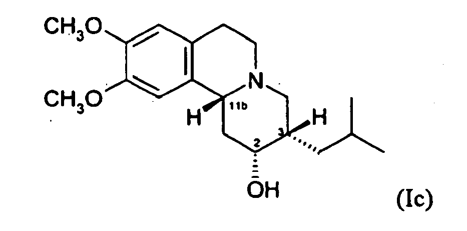 and
(d) the 2*S*,3*R*,11b*S* isomer of 3,11b-*cis*-dihydrotetrabenazine having the formula (Id): 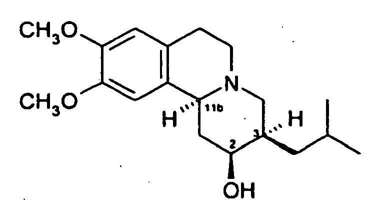 PCT/GB2005/00464 contains experimental data showing that.*cis-*dihydrotetrabenazine isomers bind to sigma-1 and sigma-2 receptors but does not disclose any therapeutic uses making use of the sigma receptor binding activity.

### Summary of the Invention

The present invention relates to the use of one of the *cis*-dihydrotetrabenazines described in our earlier application number PCT/GB2005/000464 in the treatment of inflammatory diseases.

Accordingly, in a first aspect, the invention provides a 3,11b-*cis*-dihydrotetrabenazine compound of the formula (Ia): 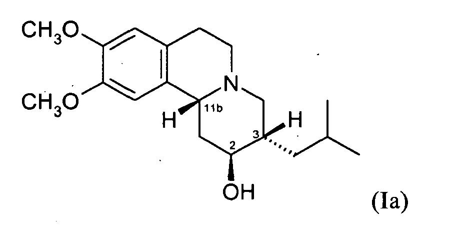 or a pharmaceutically acceptable salt thereof, for use in the prophylaxis or treatment of an inflammatory disease.

The invention also provides the use of a compound of the formula (Ia) or a pharmaceutically acceptable salt thereof as hereinbefore defined for the manufacture of a medicament for the prophylaxis or treatment of an inflammatory disease.

The compound of formula (Ia) is also referred to herein as Isomer B.

Examples of inflammatory diseases and conditions include, but are not limited to, rheumatoid arthritis, osteoarthritis, traumatic arthritis, gouty arthritis, rubella arthritis, psoriatic arthritis, and other arthritic conditions; acute or chronic inflammatory disease states such as the inflammatory reaction induced by endotoxin or inflammatory bowel disease; Reiter's syndrome, gout, rheumatoid spondylitis, chronic pulmonary inflammatory disease, Crohn's disease and ulcerative colitis.

Particular inflammatory diseases and conditions are those that are sensitive to sigma receptor ligands, for example, sigma receptor antagonists.

One particular inflammatory disease is rheumatoid arthritis.

The 3,11b-*cis*-dihydrotetrabenazine used in the invention may be in substantially pure form, for example at an isomeric purity of greater than 90%, typically greater than 95% and more preferably greater than 98%.

The term "isomeric purity" in the present context refers to the amount of the 3,11b-*cis*-dihydrotetrabenazine present relative to the total amount or concentration of dihydrotetrabenazine of all isomeric forms. For example, if 90% of the total dihydrotetrabenazine present in the composition is the 3,1b-*cis-*dihydrotetrabenazine, then the isomeric purity is 90%.

The 11b-*cis*-dihydrotetrabenazine used in the invention may be in the form of a composition which is substantially free of 3,11b-*trans*-dihydrotetrabenazine, preferably containing less than 5% of 3,11b-*trans*-dihydrotetrabenazine, more preferably less than 3% of 3,11b-*trans*-dihydrotetrabenazine, and most preferably less than 1% of 3,11b-*trans*-dihydrotetrabenazine.

The term "3,11b-*cis*-" as used herein means that the hydrogen atoms at the 3- and 11b-positions of the dihydrotetrabenazine structure are in the *cis* relative orientation.

The compound of formula (Ia) (Isomer B) may be presented in a substantially enantiomerically pure form or as a mixture with other enantiomers of 3,11 b-*cis-*dihydrotetrabenazine.

The terms "enantiomeric purity" and "enantiomerically pure" in the present context refer to the amount of Isomer B present relative to the total amount or concentration of dihydrotetrabenazine of all enantiomeric and isomeric forms. For example, if 90% of the total dihydrotetrabenazine present in the composition is in the form of Isomer B, then the enantiomeric purity is 90%.

By way of example, in each aspect and embodiment of the invention, Isomer B may be present in an enantiomeric purity of at least 55% (e.g. at least 60%, 65%, 70%, 75%, 80%, 85%, 90%, 95%, 97%, 98%, 99%, 99.5% or 100%).

The isomer of the invention may also be presented in the form of mixtures of Isomer B and one or more of Isomers A, C and D. Such mixtures may be racemic mixtures or non-racemic mixtures. Examples of racemic mixtures include the racemic mixture of Isomer A and Isomer B.

### Pharmaceutically Acceptable Salts

Unless the context requires otherwise, a reference in this application to the compound of formula (Ia) or Isomer B includes within its scope not only the free base of the compound but also its salts, and in particular acid addition salts.

Particular acids from which the acid addition salts are formed include acids having a pKa value of less than 3.5 and more usually less than 3. For example, the acid addition salts can be formed from an acid having a pKa in the range from +3.5 to -3:5.

Preferred acid addition salts include those formed with sulphonic acids such as methanesulphonic acid, ethanesulphonic acid, benzene sulphonic acid, toluene sulphonic acid, camphor sulphonic acid and naphthalene sulphonic acid.

One particular acid from which acid addition salts may be formed is methanesulphonic acid.

Acid addition salts can be prepared by the methods described herein or conventional chemical methods such as the methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free base form of the compound with the appropriate base or acid in water or in an organic solvent, or in a mixture of the two; generally, nonaqueous media such as ether, ethyl acetate, ethanol, isopropanol, or acetonitrile are used.

The salts are typically pharmaceutically acceptable salts. However, salts that are not pharmaceutically acceptable may also be prepared as intermediate forms which may then be converted into pharmaceutically acceptable salts. Such non-pharmaceutically acceptable salt forms also form part of the invention.

### Methods for the Preparation of Dihydrotetrabenazine Isomers

In this section, methods for preparing all four 3,11b-*cis*-dihydrotetrabenazine isomers are described although the invention relates only to the therapeutic uses of the compound of formula (Ia) (Isomer B).

The 3,11b-*cis*-dihydrotetrabenazines can be prepared by a process comprising the reaction of a compound of the formula (II): 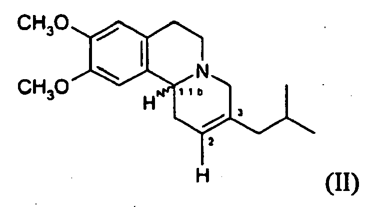 with a reagent or reagents suitable for hydrating the 2,3-double bond in the compound of formula (II) and thereafter where required separating and isolating a desired dihydrotetrabenazine isomer form.

The hydration of the 2,3-double bond can be carried out by hydroboration using a borane reagent such as diborane or a borane-ether (e.g. borane-tetrahydrofuran (THF)) to give an intermediate alkyl borane adduct followed by oxidation of the alkyl borane adduct and hydrolysis in the presence of a base. The hydroboration is typically carried out in a dry polar non-protic solvent such as an ether (e.g. THF), usually at a non-elevated temperature, for example room temperature. The borane-alkene adduct is typically oxidised with an oxidising agent such as hydrogen peroxide in the presence of a base providing a source of hydroxide ions, such as ammonium hydroxide or an alkali metal hydroxide, e.g. potassium hydroxide or sodium hydroxide. The hydroboration-oxidation-hydrolysis sequence of reactions of Process A typically provides dihydrotetrabenazine isomers in which the hydrogen atoms at the 2- and 3-positions have a *trans* relative orientation.

Compounds of the formula (II) can be prepared by reduction of tetrabenazine to give a dihydrotetrabenazine followed by dehydration of the dihydrotetrabenazine. Reduction of the tetrabenazine can be accomplished using an aluminium hydride reagent such as lithium aluminium hydride, or a borohydride reagent such as sodium borohydride, potassium borohydride or a borohydride derivative, for example an alkyl borohydride such as lithium tri-sec-butyl borohydride. Alternatively, the reduction step can be effected using catalytic hydrogenation, for example over a Raney nickel or platinum oxide catalyst. Suitable conditions for performing the reduction step are described in more detail below or can be found in US 2,843,591 (Hoffmann- La Roche) and Brossi et al., Helv. Chim. Acta., vol. XLI, No. 193, pp1793-1806 (1958).

Because the tetrabenazine used as the starting material for the reduction reaction is typically a mixture of the *RR* and *SS* isomers (i.e. *trans*-tetrabenazine), the dihydrotetrabenazine formed by the reduction step will have the same *trans* configuration about the 3- and 11b positions and will take the form of one or more of the known dihydrotetrabenazine isomers shown in Figure 3 above. Thus Process A may involve taking the *trans* isomers of dihydrotetrabenazine, dehydrating them to form the alkene (II) and then "rehydrating" the alkene (II) using conditions that give the required *cis* dihydrotetrabenazine isomers.

Dehydration of the dihydrotetrabenazine to the alkene (II) can be carried out using a variety of standard conditions for dehydrating alcohols to form alkenes, see for example J. March (*idem*) pages 389-390 and references therein. Examples of such conditions include the use of phosphorus-based dehydrating agents such as phosphorus halides or phosphorus oxyhalides, e.g. POCl₃ and PCl_{5.} As an alternative to direct dehydration, the hydroxyl group of the dihydrotetrabenazine can be converted to a leaving group L such as halogen (e.g. chlorine or bromine) and then subjected to conditions (e.g. the presence of a base) for eliminating H-L. Conversion of the hydroxyl group to a halide can be achieved using methods well known to the skilled chemist, for example by reaction with carbon tetrachloride or carbon tetrabromide in the presence of a trialkyl or triaryl phosphine such as triphenyl phosphine or tributyl phosphine.

The tetrabenazine used as the starting material for the reduction to give the dihydrotetrabenazine can be obtained commercially or can be synthesised by the method described in US 2,830,993 (Hoffmann-La Roche).

Another process (Process B) for preparing a 3,11b-*cis*-dihydrotetrabenazine compound comprises subjecting a compound of the formula (III): 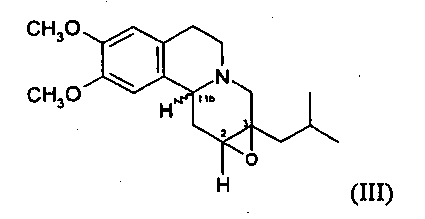 to conditions for ring-opening the 2,3-epoxide group in the compound of the formula (III), and thereafter where required separating and isolating a desired dihydrotetrabenazine isomer form.

The ring-opening can be effected in accordance with known methods for epoxide ring openings. However, a currently preferred method of ring-opening the epoxide is reductive ring opening which can be achieved using a reducing agent such as borane-THF. Reaction with borane-THF can be carried out in a polar non-protic solvent such as ether (e.g. tetrahydrofuran) usually at ambient temperature, the borne complex thus formed being subsequently hydrolysed by heating in the presence of water and a base at the reflux temperature of the solvent. Process B typically gives rise to dihydrotetrabenazine isomers in which the hydrogen atoms at the 2- and 3-positions have a *cis* relative orientation.

The epoxide compounds of the formula (III) can be prepared by epoxidation of an alkene of the formula (II) above. The epoxidation reaction can be carried out using conditions and reagents well known to the skilled chemist, see for example J. March (*idem*), pages 826-829 and references therein. Typically, a per-acid such as meta-chloroperbenzoic acid (MCPBA), or a mixture of a per-acid and a further oxidising agent such as perchloric acid, may be used to bring about epoxidation.

When the starting materials for processes A and B above are mixtures of enantiomers, then the products of the processes will typically be pairs of enantiomers, for example racemic mixtures, possibly together with diastereoisomeric impurities. Unwanted diastereoisomers can be removed by techniques such as chromatography (e.g. HPLC) and the individual enantiomers can be separated by a variety of methods known to the skilled chemist. For example, they can be separated by means of:
(i) chiral chromatography (chromatography on a chiral support); or
(ii) forming a salt with an optically pure chiral acid, separating the salts of the two diastereoisomers by fractional crystallisation and then releasing the dihydrotetrabenazine from the salt; or
(iii) forming a derivative (such as an ester) with an optically pure chiral derivatising agent (e.g. esterifying agent), separating the resulting epimers (e.g. by chromatography) and then converting the derivative to the dihydrotetrabenazine.

One method of separating pairs of enantiomers obtained from each of Processes A and B, and which has been found to be particularly effective, is to esterify the hydroxyl group of the dihydrotetrabenazine with an optically active form of Mosher's acid, such as the *R* (+) isomer shown below, or an active form thereof:

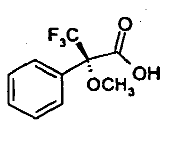

The resulting esters of the two enantiomers of the dihydrobenazine can then be separated by chromatography (e.g. HPLC) and the separated esters hydrolysed to give the individual dihydrobenazine isomers using a base such as an alkali metal hydroxide (e.g. NaOH) in a polar solvent such as methanol.

As an alternative to using mixtures of enantiomers as the starting materials in processes A and B and then carrying out separation of enantiomers subsequently, processes A and B can each be carried out on single enantiomer starting materials leading to products in which a single enantiomer predominates. Single enantiomers of the alkene (II) can be prepared by subjecting RR/SS tetrabenazine to a stereoselective reduction using lithium tri-*sec*-butyl borohydride to give a mixture of SRR and RSS enantiomers of dihydrotetrabenazine, separating the enantiomers (e.g. by fractional crystallisation) and then dehydrating a separated single enantiomer of dihydrotetrabenazine to give predominantly or exclusively a single enantiomer of the compound of formula (II).

Processes A and B are illustrated in more detail below in Schemes 1 and 2 respectively.

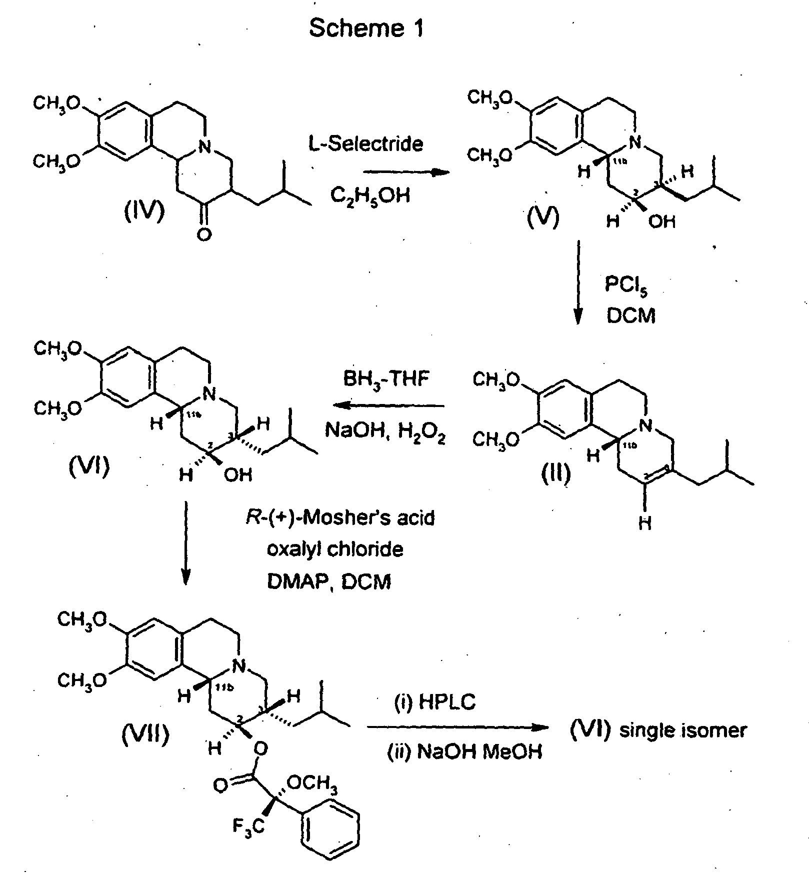

Scheme 1 illustrates the preparation of individual dihydrotetrabenazine isomers having the 2*S*,3*S*,11b*R* and 2*R*,3*R*,11b*S* configurations in which the hydrogen atoms attached to the 2- and 3-positions are arranged in a *trans* relative orientation. This reaction scheme includes Process A defined above.

The starting point for the sequence of reactions in Scheme 1 is commercially available tetrabenazine (IV) which is a racemic mixture of the RR and SS optical isomers of tetrabenazine. In each of the RR and SS isomers, the hydrogen atoms at the 3- and 11b-positions are arranged in a *trans* relative orientation. As an alternative to using the commercially available compound, tetrabenazine can be synthesised according to the procedure described in US patent number 2,830,993 (see in particular example 11).

The racemic mixture of RR and SS tetrabenazine is reduced using the borohydride reducing agent lithium tri-sec-butyl borohydride ("L-Selectride") to give a mixture of the known 2*S*,3*R*,11b*R* and 2*R*,3*S*,11b*S* isomers (V) of dihydrotetrabenazine, of which only the 2*S*,3*R*,11b*R* isomer is shown for simplicity. By using the more sterically demanding L-Selectride as the borohydride reducing agent rather than sodium borohydride, formation of the RRR and SSS isomers of dihydrotetrabenazine is minimised or suppressed.

The dihydrotetrabenazine isomers (V) are reacted with a dehydrating agent such as phosphorus pentachloride in a non-protic solvent such as a chlorinated hydrocarbon (for example chloroform or dichloromethane, preferably dichloromethane) to form the unsaturated compound (II) as a pair of enantiomers, only the *R*-enantiomer of which is shown in the Scheme. The dehydration reaction is typically carried out at a temperature lower than room temperature, for example at around 0-5°C.

The unsaturated compound (II) is then subjected to a stereoselective re-hydration to generate the dihydrotetrabenazine (VI) and its mirror image or antipode (not shown) in which the hydrogen atoms at the 3- and 11b-positions are arranged in a *cis* relative orientation and the hydrogen atoms at the 2- and 3-positions are arranged in a *trans* relative orientation. The stereoselective rehydration is accomplished by a hydroboration procedure using borane-THF in tetrahydrofuran (the) to form an intermediate borane complex (not shown) which is then oxidised with hydrogen peroxide in the presence of a base such as sodium hydroxide.

An initial purification step may then be carried out (e.g. by HPLC) to give the product (V) of the rehydration reaction sequence as a mixture of the 2*S*,3*S*,11b*R* and 2*R*,3*R*,11b*S* isomers of which only the 2*S*,3*S*,11b*R* isomer is shown in the Scheme. In order to separate the isomers, the mixture is treated with *R* (+) Mosher's acid, in the presence of oxalyl chloride and dimethylaminopyridine (DMAP) in dichloromethane to give a pair of diastereoisomeric esters (VII) (of which only one diastereoisomer is shown) which can then be separated using HPLC. The individual esters can then be hydrolysed using an alkali metal hydroxide such as sodium hydroxide to give a single isomer (VI).

In a variation of the sequence of steps shown in Scheme 1, following the reduction of RR/SS tetrabenazine, the resulting mixture of enantiomers of the dihydrotetrabenazine (V) can be separated to give the individual enantiomers. Separation can be carried out by forming a salt with a chiral acid such as (+) or (-) camphorsulphonic acid, separating the resulting diastereoisomers by fractional crystallisation to give a salt of a single enantiomer and then releasing the free base from the salt.

The separated dihydrotetrabenazine enantiomer can be dehydrated to give a single enantiomer of the alkene (II). Subsequent rehydration of the alkene (II) will then give predominantly or exclusively a single enantiomer of the cis-dihydrotetrabenazine (VI). An advantage of this variation is that it does not involve the formation of Mosher's acid esters and therefore avoids the chromatographic separation typically used to separate Mosher's acid esters.

Scheme 2 illustrates the preparation of individual dihydrotetrabenazine isomers having the 2*R*,3*S*,11b*R* and 2*S*,3*R*,11b*S* configurations in which the hydrogen atoms attached to the 2- and 3-positions are arranged in a *cis* relative orientation. This reaction scheme includes Process B defined above.

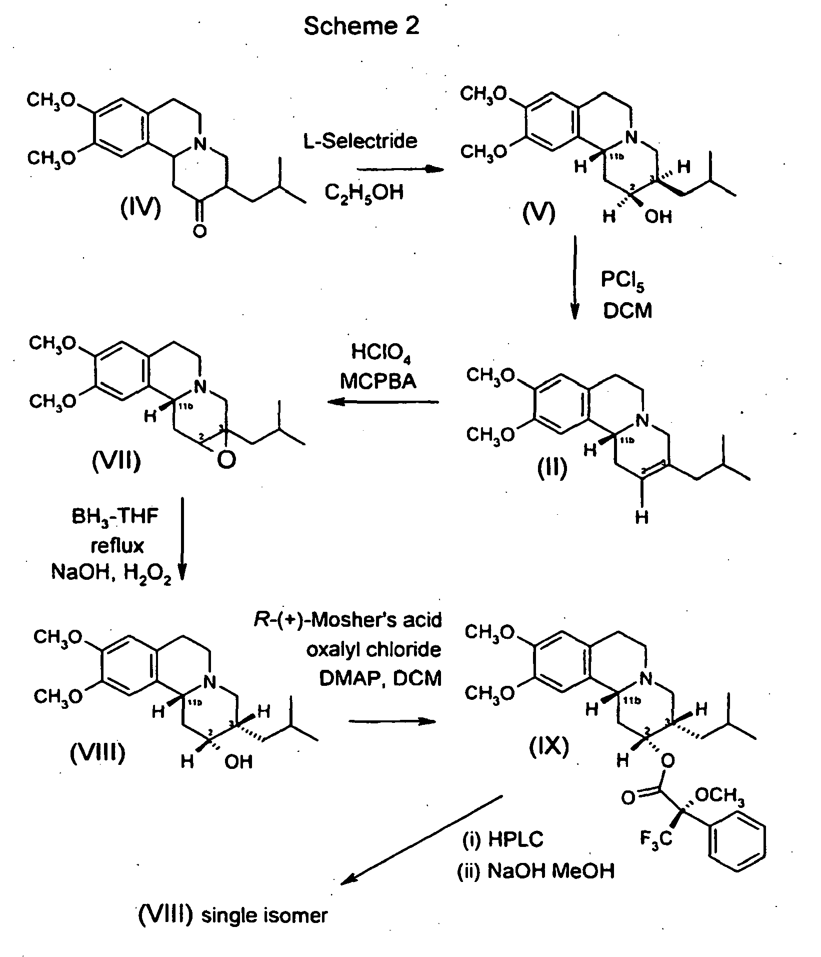

In Scheme 2, the unsaturated compound (II) is produced by reducing tetrabenazine to give the 2S,3R, 11 bR and 2R,3S,11bS isomers (V) of dihydrotetrabenazine and dehydrating with PCl₅ in the manner described above in Scheme 1. However, instead of subjecting the compound (II) to hydroboration, the 2,3-double bond is converted to an epoxide by reaction with *meta*-chloroperbenzoic acid (MCPBA) and perchloric acid. The epoxidation reaction is conveniently carried out in an alcohol solvent such as methanol, typically at around room temperature.

The epoxide (VII) is then subjected to a reductive ring opening using borane-THF as an electrophilic reducing agent to give an intermediate borane complex (not shown) which is then oxidised and cleaved with hydrogen peroxide in the presence of an alkali such as sodium hydroxide to give a dihydrotetrabenazine (VIII) as a mixture of the 2*R*,3*S*,11b*R* and 2*S*,3*R*,11b*S* isomers, of which only the 2*R*,3*S*,11b*R* is shown for simplicity. Treatment of the mixture of isomers (VIII) with *R* (+) Mosher's acid in the presence of oxalyl chloride and dimethylaminopyridine (DMAP) in dichloromethane gives a pair of epimeric esters (IX) (of which only one epimer is shown) which can then by separated by chromatography and hydrolysed with sodium hydroxide in methanol in the manner described above in relation to Scheme 1.

### Biological Activity

The four *cis*-dihydrotetrabenazine isomers bind to both sigma-1 and sigma-2 receptors, as illustrated in the examples below. The four isomers are approximately equipotent as ligands for sigma-2 but isomers B and D bind more strongly to the sigma-1 receptor than do isomers A and C.

It is envisaged that the *cis*-dihydrotetrabenazine isomers will be active as anti-inflammatory agents. The anti-inflammatory activities of the compounds can be tested using a variety of methods well known to the skilled person.

The ability of the compounds to treat arthritis can be demonstrated in any one or more of the following test assays and models:
(i) A murine collagen-induced arthritis model as described in Kakimoto et al., Cell Immunol. 142: 326-337, 1992.
(ii) A rat collagen-induced arthritis model as described in Knoerzer et al., Toxicol. Pathol. 25:13-19, 1997.
(iii) A rat adjuvant arthritis model as described in Halloran et al., Arthritis Rheum. 39: 810-819, 1996.
(iv) A rat streptococcal cell wall-induced arthritis model as described in Schimmer, et al., J. Immunol. 160: 1466-1477, 1998.
(v) A SCID-mouse human rheumatoid arthritis model as described in Oppenheimer-Marks et al., J. Clin. Invest. 101: 1261-1272, 1998.

The ability of the compounds to treat inflammatory lung injury can be demonstrated by the following assays and test models:
(vi) A murine oxygen-induced lung injury model as described in Wegner et al., Lung 170:267-279, 1992.
(vii) A murine immune complex-induced lung injury model as described in Mulligan et al., J. Immunol. 154:1350-1363, 1995.
(viii) A murine acid-induced lung injury model as described in Nagase et al., Am. J. Respir. Crit. Care Med. 154:504-510, 1996.

The ability of the compounds to treat inflammatory bowel disease can be demonstrated in a rabbit chemical-induced colitis model as described in Bennet et al., J. Pharmaco/. Exp. Ther. 280:988-1000, 1997.

The ability of the compounds to treat inflammatory liver injury can be demonstrated in a murine liver injury model as described in Tanaka et al., J. Immunol. 151:5088-5095, 1993.

The ability of the compounds to treat inflammatory glomerular injury can be demonstrated in a rat nephrotoxic serum nephritis model as described in Kawasaki, et al., J. Immunol. 150:1074-1083, 1993.

The anti-inflammatory activity of the compounds is also indicated by its ability to reduce the production of pro-inflammatory cytokines and inhibit T-cell proliferation as described in the Examples below.

### Pharmaceutical Formulations

The compound of formula (Ia) is typically administered in the form of a pharmaceutical composition.

The pharmaceutical compositions can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules, caplets, pills, lozenges, syrups, solutions, sprays, powders, granules, elixirs and suspensions, sublingual tablets, sprays, wafers or patches and buccal patches.

Pharmaceutical compositions containing the dihydrotetrabenazine compound of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as com starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (e.g.; tablets, capsules etc.) can be coated or un-coated, but typically have a coating, for example a protective film coating (e.g. a wax or varnish) or a release controlling coating. The coating (e.g. a Eudragit™ type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the inventions will generally be presented in unit dosage form and, as such, will typically contain sufficient compound to provide a desired level of biological activity. For example, a formulation intended for oral administration may contain from 2 milligrams to 200 milligrams of active ingredient, more usually from 10 milligrams to 100 milligrams, for example, 12.5 milligrams, 25 milligrams and 50 milligrams.

The active compound will be administered to a patient in need thereof (for example a human or animal patient) in an amount sufficient to achieve the desired therapeutic effect.

The subject in need of such administration is typically a patient suffering from or at risk of suffering from an inflammatory disease as hereinbefore defined.

The compounds will typically be administered in amounts that are therapeutically or prophylactically useful and which generally are non-toxic. However, in certain situations, the benefits of administering the dihydrotetrabenazine compound may outweigh the disadvantages of any toxic effects or side effects, in which case it may be considered desirable to administer compounds in amounts that are associated with a degree of toxicity.

A typical daily dose of the compound can be up to 1000 mg per day, for example in the range from 0.01 milligrams to 10 milligrams per kilogram of body weight, more usually from 0.025 milligrams to 5 milligrams per kilogram of body weight, for example up to 3 milligrams per kilogram of bodyweight, and more typically 0.15 milligrams to 5 milligrams per kilogram of bodyweight although higher or lower doses may be administered where required.

### BRIEF DESCRIPTION OF THE DRAWINGS

Figure 1 illustrates the effect of the compounds Isomer B (RU350) and Isomer D (RU346) on the production of TNFα by monocytes.

Figure 2 illustrates the effect of the compounds Isomer B (RU350) and Isomer D (RU346) on the production of IL-4 by monocytes.

Figure 3 illustrates the effect of the compounds Isomer B (RU350) and Isomer D (RU346) on the production of IL-2 by human monocytes.

Figure 4 illustrates the effect of the compounds Isomer B (RU350) and Isomer D (RU346) on the production of IL-5 by human monocytes.

Figure 5 illustrates the effect of the compounds Isomer B (RU350) and Isomer D (RU346) on the production of IL-10 by human monocytes.

Figure 6 illustrates the effect of the compounds Isomer B (RU350) and Isomer D (RU346) on the production ofIL-12 by human monocytes.

### EXAMPLES

The following non-limiting examples illustrate the synthesis and properties of the 3,11 b-*cis*-dihydrotetrabenazine isomers. The examples describe all four isomers of 3,11b-*cis*-dihydrotetrabenazine although the invention is limited to the therapeutic uses of Isomer B (the compound of formula (Ia)). The examples relating to the other isomers are retained as comparative examples.

### EXAMPLE 1

### Preparation of 2S,3S,11bR and 2R,3R,11bS Isomers of Dihydrotetrabenazine

### 1A. Reduction of RR/SS Tetrabenazine

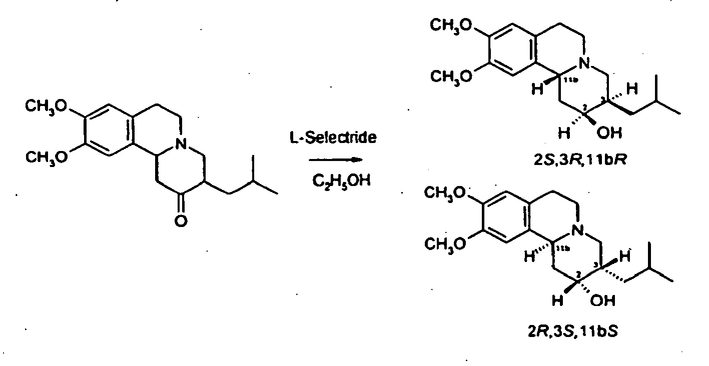

1M L-Selectride^{®} in tetrahydrofuran (135 ml, 135 mmol, 2.87 eq.) was added slowly over 30 minutes to a stirred solution of tetrabenazine *RR*/SS racemate (15 g, 47 mmol) in ethanol (75 ml) and tetrahydrofuran (75 ml) at 0 °C. After addition was complete the mixture was stirred at 0 °C for 30 minutes and then allowed to warm to room temperature.

The mixture was poured onto crushed ice (300 g) and water (100 ml) added. The solution was extracted with diethyl ether (2 x 200 ml) and the combined ethereal extracts washed with water (100 ml) and partly dried over anhydrous potassium carbonate. Drying was completed using anhydrous magnesium sulphate and, after filtration, the solvent was removed at reduced pressure (shielded from the light, bath temperature <20 °C) to afford a pale yellow solid.

The solid was slurried with petroleum ether (30-40 °C) and filtered to afford a white powdery solid (12 g, 80%).

### 1B. Dehydration of reduced Tetrabenazine

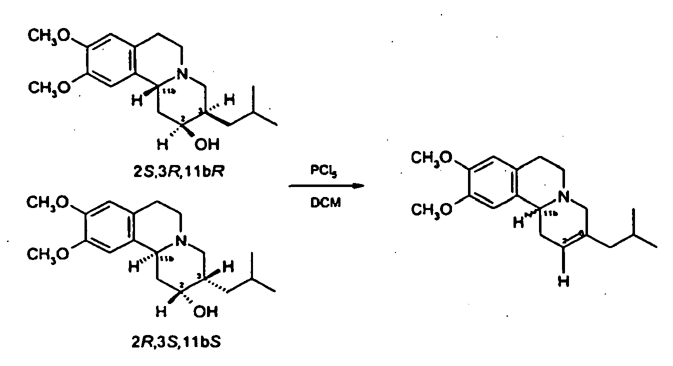

Phosphorous pentachloride (32.8 g, 157.5 mmol, 2.5 eq) was added in portions over 30 minutes to a stirred solution of the reduced tetrabenazine product from Example 1A (20 g, 62.7 mmol) in dichloromethane (200 ml) at 0 °C. After the addition was complete, the reaction mixture was stirred at 0 °C for a further 30 minutes and the solution poured slowly into 2M aqueous sodium carbonate solution containing crushed ice (0 °C). Once the initial acid gas evolution had ceased the mixture was basified (ca. pH 12) using solid sodium carbonate.

The alkaline solution was extracted using ethyl acetate (800 ml) and the combined organic extracts dried over anhydrous magnesium sulphate. After filtration the solvent was removed at reduced pressure to afford a brown oil, which was purified by column chromatography (silica, ethyl acetate) to afford the semi-pure alkene as a yellow solid (10.87 g, 58%).

### 1C. Hydration of the Crude Alkene from Example 1B

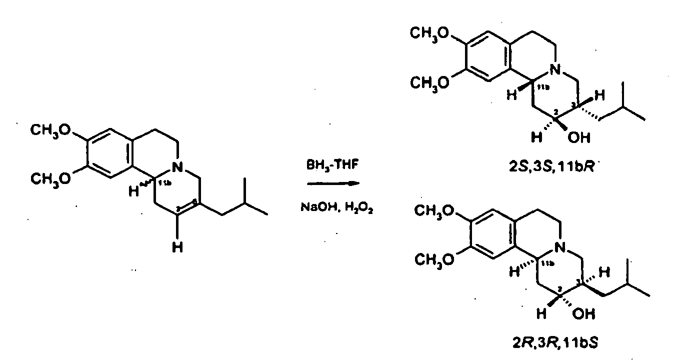

A solution of the crude alkene (10.87 g, 36.11 mmol) from Example 1B in dry THF (52 ml) at room temperature was treated with 1M borane-THF (155.6 ml, 155.6 mmol, 4.30 eq) added in a dropwise manner. The reaction was stirred for 2 hours, water (20 ml) was added and the solution basified to pH 12 with 30% aqueous sodium hydroxide solution.

Aqueous 30% hydrogen peroxide solution (30 ml) was added to the stirred alkaline reaction mixture and the solution was heated to reflux for 1 hour before being allowed to cool. Water (100 ml) was added and the mixture extracted with ethyl acetate (3 x 250 ml). The organic extracts were combined and dried over anhydrous magnesium sulphate and after filtration the solvent was removed at reduced pressure to afford a yellow oil (9 g).

The oil was purified using preparative HPLC (Column: Lichrospher Si60, 5 µm, 250 x 21.20 mm, mobile phase: hexane : ethanol : dichloromethane (85:15:5); UV 254 nm, flow: 10 ml min⁻¹) at 350 mg per injection followed by concentration of the fractions of interest under vacuum. The product oil was then dissolved in ether and concentrated once more under vacuum to give the dihydrotetrabenazine racemate shown above as a yellow foam (5.76 g, 50%).

### 1D. Preparation of Mosher's ester derivatives

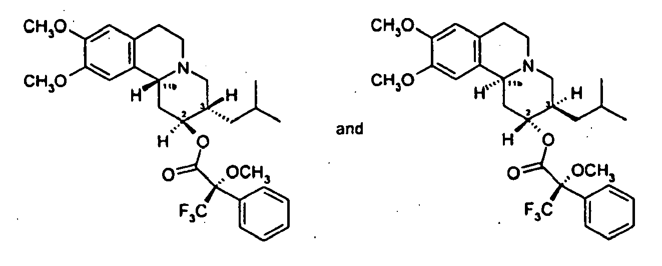

R-(+)-α-methoxy-α-trifluoromethylphenyl acetic acid (5 g, 21.35 mmol), oxalyl chloride (2.02 ml) and DMF (0.16 ml) were added to anhydrous dichloromethane (50 ml) and the solution was stirred at room temperature for 45 minutes. The solution was concentrated under reduced pressure and the residue was taken up in anhydrous dichloromethane (50 ml) once more. The resulting solution was cooled using an ice-water bath and dimethylaminopyridine (3.83 g, 31.34 mmol) was added followed by a pre-dried solution (over 4Å sieves) in anhydrous dichloromethane of the solid product of Example 1C (5 g, 15.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (10:1)). Evaporation of the collected column fractions of interest and removal of the solvent at reduced pressure gave a solid which was further purified using column chromatography (silica, hexane : ethyl acetate (1:1)) to give three main components which were partially resolved into Mosher's ester peaks 1 and 2.

Preparative HPLC of the three components (Column: 2 x Lichrospher Si60, 5 µm, 250 x 21.20 mm, mobile phase: hexane : isopropanol (97:3), UV 254 nm; flow: 10 ml min⁻¹) at 300 mg loading followed by concentration of the fractions of interest under vacuum gave the pure Mosher's ester derivatives
Peak 1 (3.89 g, 46.5%)
Peak 2 (2.78 g, 33%)

The fractions corresponding to the two peaks were subjected to hydrolysis to liberate the individual dihydrotetrabenazine isomers identified and characterised as Isomers A and B. Isomers A and B are each believed to have one of the following structures

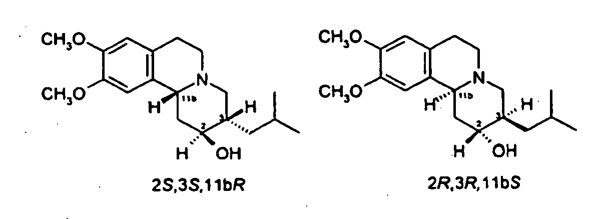

More specifically, Isomer B is believed to have the 2*S*,3*S*,11b*R* absolute configuration on the basis of the X-ray crystallography experiments described in Example 4 below.

### 1E. Hydrolysis of Peak 1 to give Isomer A

Aqueous 20% sodium hydroxide solution (87.5 ml) was added to a solution of Mosher's ester peak 1 (3.89 g, 7.27 mmol) in methanol (260 ml) and the mixture stirred and heated to reflux for 150 minutes. After cooling to room temperature water (200 ml) was added and the solution extracted with ether (600 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure to give a yellow foam. This material was purified by column chromatography (silica, gradient elution of ethyl acetate : hexane (1:1) to ethyl acetate). The fractions of interest were combined and the solvent removed at reduced pressure. The residue was taken up in ether and the solvent removed at reduced pressure once more to give Isomer A as an off-white foam (1.1g, 47%).

Isomer A, which is believed to have the 2*R*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for isomer A are set out in Table 1 and the Chiral HPLC and ORD data are set out in Table 3.

### 1F. Hydrolysis of Peak 2 to give Isomer B

Aqueous 20% sodium hydroxide solution (62.5 ml) was added to a solution of Mosher's ester peak 2 (2.78 g, 5.19 mmol) in methanol (185 ml) and the mixture stirred and heated to reflux for 150 minutes. After cooling to room temperature water (142 ml) was added and the solution extracted with ether (440 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure. Petroleum ether (30-40 °C) was added to the residue and the solution concentrated under vacuum once more to give Isomer B as a white foam (1.34 g, 81%).

Isomer B, which is believed to have the 2*S*,3*S*,11b*R* configuration, was characterized by ¹H-NMR, ¹³C-NMR, IR, mass spectrometry, chiral HPLC, ORD and X-ray crystallography. The IR, NMR and MS data for Isomer B are set out in Table 1 and the Chiral HPLC and ORD data are set out in Table 3. The X-ray crystallography data are set out in Example 4.

### EXAMPLE 2

### Preparation of 2R,3S,11bR and 2S,3R,11bS Isomers of Dihydrotetrabenazine

### 2A. Preparation of 2,3-Dehydrotetrabenazine

A solution containing a racemic mixture (15 g, 47 mmol) of *RR* and *SS* tetrabenazine enantiomers in tetrahydrofuran was subjected to reduction with L-Selectride^{®} by the method of Example 1A to give a mixture of the 2*S*,3*R*,11b*R* and 2*R*,3*S*,11b*S* enantiomers of dihydrotetrabenazine.as a white powdery solid'(1 2 g, 80%). The partially purified dihydrotetrabenazine was then dehydrated using PCl₅ according to the method of Example 1B to give a semi-pure mixture of 11b*R* and 11b*S* isomers of 2,3-dehydrotetrabenazine (the 11b*R* enantiomer of which is shown below) as a yellow solid (12.92 g, 68%).

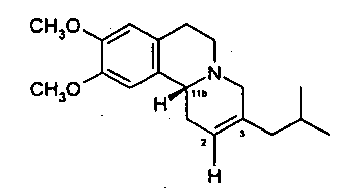

### 2B.Epoxidation of the Crude Alkene from Example 2A

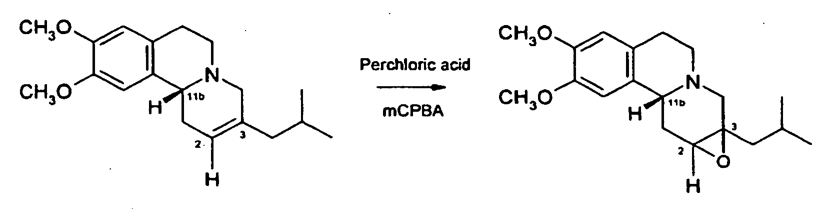

To a stirred solution of the crude alkene from Example 2A (12,92 g, 42.9 mmol) in methanol (215 ml) was added a solution of 70% perchloric acid (3.70 ml, 43 mmol) in methanol (215 ml). 77% 3-Chloroperoxybenzoic acid (15.50 g, 65 mmol) was added to the reaction and the resulting mixture was stirred for 18 hours at room temperature protected from light.

The reaction mixture was poured into saturated aqueous sodium sulphite solution (200 ml) and water (200 ml) added. Chloroform (300 ml) was added to the resulting emulsion and the mixture basified with saturated aqueous sodium bicarbonate (400 ml).

The organic layer was collected and the aqueous phase washed with additional chloroform (2 x 150 ml). The combined chloroform layers were dried over anhydrous magnesium sulphate and after filtration the solvent was removed at reduced pressure to give a brown oil (14.35 g, yield > 100% - probable solvent remains in product). This material was used without further purification.

### 2C. Reductive Ring Opening of the Epoxide from 2B

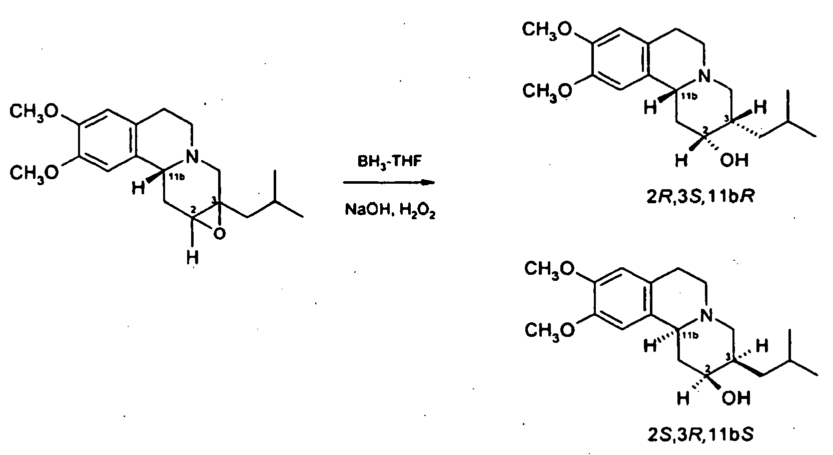

A stirred solution of the crude epoxide from Example 2B (14.35 g, 42.9 mmol, assuming 100% yield) in dry THF (80 ml) was treated slowly with 1M borane/THF (184.6 ml, 184.6 mmol) over 15 minutes. The reaction was stirred for two hours, water (65 ml) was added and the solution heated with stirring to reflux for 30 minutes.

After cooling, 30% sodium hydroxide solution (97 ml) was added to the reaction mixture followed by 30% hydrogen peroxide solution (48.6 ml) and the reaction was stirred and heated to reflux for an additional 1 hour.

The cooled reaction mixture was extracted with ethyl acetate (500 ml) dried over anhydrous magnesium sulphate and after filtration the solvent was removed at reduced pressure to give an oil. Hexane (230 ml) was added to the oil and the solution re-concentrated under reduced pressure.

The oily residue was purified by column chromatography (silica, ethyl acetate). The fractions of interest were combined and the solvent removed under reduced pressure. The residue was purified once more using column chromatography (silica, gradient, hexane to ether). The fractions of interest were combined and the solvents evaporated at reduced pressure to give a pale yellow solid (5.18 g, 38%).

### 2D. Preparation of Mosher's ester derivatives of the 2R,3S,11bR and 2S,3R,11bS Isomers of Dihydrotetrabenazine

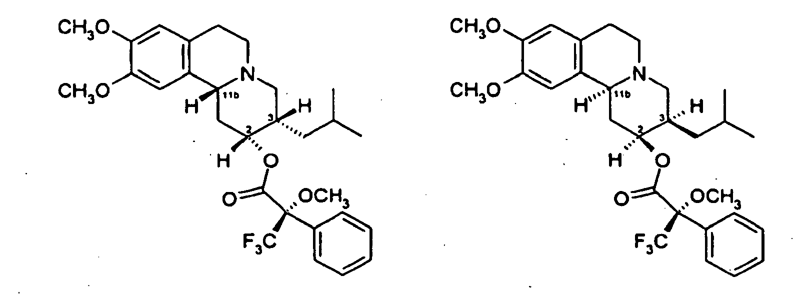

R-(+)-α-methoxy-α-trifluoromethylphenyl acetic acid (4.68 g, 19.98 mmol), oxalyl chloride (1.90 ml) and DMF (0.13 ml) were added to anhydrous dichloromethane (46 ml) and the solution stirred at room temperature for 45 minutes. The solution was concentrated under reduced pressure and the residue was taken up in anhydrous dichloromethane (40 ml) once more. The resulting solution was cooled using an ice-water bath and dimethylaminopyridine (3.65 g, 29.87 mmol) was added followed by a pre-dried solution (over 4Å sieves) in anhydrous dichloromethane (20 ml) of the solid product of Example 2C (4.68 g, 14.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (1:1)). Evaporation of the collected column fractions of interest and removal of the solvent at reduced pressure gave a pink solid (6.53 g)

Preparative HPLC of the solid (Column: 2 x Lichrospher Si60, 5 µm, 250 x 21.20 mm; mobile phase hexane : isopropanol (97:3); UV 254 nm; flow: 10 ml min⁻¹) at 100 mg loading followed by concentration of the fractions of interest under vacuum gave a solid which was slurried with petroleum ether (30-40 °C) and collected by filtration to give the pure Mosher's ester derivatives
Peak 1 (2.37 g, 30%)
Peak 2 (2.42 g, 30%)

The fractions corresponding to the two peaks were subjected to hydrolysis to liberate the individual dihydrotetrabenazine isomers identified and characterised as Isomers C and D. Isomers C and D are each believed to have one of the following structures

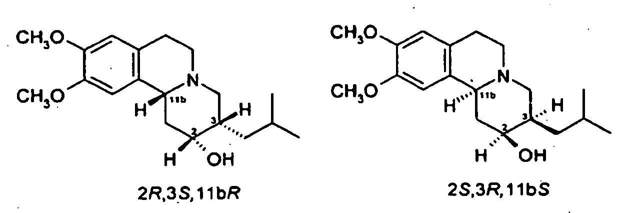

### 2F.Hydrolysis of Peak 1 to give Isomer C

20% aqueous sodium hydroxide solution (53 ml) was added to a stirred solution of Mosher's ester peak 1 (2.37 g, 4.43 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended solid collected by filtration. The filtrate was concentrated at reduced pressure and the second batch of suspended solid was collected by filtration. Both collected solids were combined and dried under reduced pressure to give Isomer C (1.0 g, 70%).

Isomer C, which is believed to have either the 2*R*,3*S*,11b*R* or 2*S*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer C are set out in Table 2 and the Chiral HPLC and ORD data are set out in Table 4.

### 2G. Hydrolysis of Peak 2 to give Isomer D

20% aqueous sodium hydroxide solution (53 ml) was added to a stirred solution of Mosher's ester peak 2 (2.42 g, 4.52 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended orange solid collected by filtration. The solid was dissolved in ethyl acetate : hexane (15:85) and purified by column chromatography (silica, gradient ethyl acetate : hexane (15:85) to ethyl acetate). The fractions of interest were combined and the solvent removed at reduced pressure. The residue was slurried with petroleum ether (30-40 °C) and the resulting suspension collected by filtration. The collected solid was dried under reduced pressure to give Isomer D as a white solid (0.93 g, 64%).

Isomer D, which is believed to have either the 2*R*,3*S*,11b*R* or 2*S*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer D are set out in Table 2 and the Chiral HPLC and ORD data are set out in Table 4.

In Tables 1 and 2, the infra red spectra were determined using the KBr disc method. The ¹H NMR spectra were carried out on solutions in deuterated chloroform using a Varian Gemini NMR spectrometer (200 MHz.). The ¹³C NMR spectra were carried out on solutions in deuterated chloroform using a Varian Gemini NMR spectrometer (50MHz). The mass spectra were obtained using a Micromass Platform II (ES⁺ conditions) spectrometer. In Tables 3 and 4, the Optical Rotatory Dispersion figures were obtained using an Optical Activity PolAAr 2001 instrument in methanol solution at 24°C. The HPLC retention time measurements were carried out using an HP 1050 HPLC chromatograph with UV detection.

**Tables 1 and 2 - Spectroscopic Data**

| Table 1 | | | | |
|---|---|---|---|---|
| Dihydrotetrabenazine isomer | ¹H-NMR spectrum (CDCl₃) | ¹³C-NMR spectrum (CDCl₃) | IR Spectrum (KBr solid) | Mass Spectrum (ES⁺) |
| Isomers A and B | 6.67 δ 1H (s); | 147.7 δ; | 2950 cm⁻¹; | MH⁺ 320 |
| 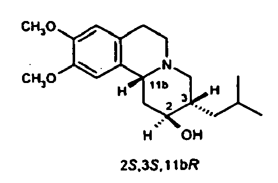 | 6.57 δ 1H (s); | 147.6 δ; | 2928 cm⁻¹; | |
| | 3.84 δ 6H (s); | 130.5 δ; | 2868 cm-¹; | |
| | 3.55 δ 1H (br. d); | 127.6 δ; | 2834 cm⁻¹; | |
| | 3.08 δ 1H (m); | 112.1 δ; | 1610 cm⁻¹; | |
| | 2.79 δ 2H(m); | 108.4 δ; | 1511 cm⁻¹; | |
| | 2.55 δ 3H (m); | 70.5 δ; | 1464 cm⁻¹ | |
| | 2.17 δ 1H (m); | 57.5 δ; | 1364 cm⁻¹; | |
| OR | 1.72 δ 6H (m); | 56.5 δ; | 1324 cm⁻¹; | |
| 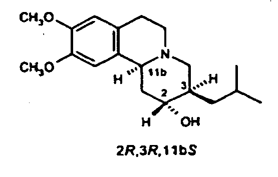 | 1.02 δ 1H (m); | 56.3 δ; | 1258 cm⁻¹; | |
| | 0.88 δ 6H (t) | 54.8 δ; | 1223 cm-¹; | |
| | | 53.2δ; | 1208 cm⁻¹; | |
| | | 40.4 δ; | 1144 cm⁻¹; | |
| | | 40.1 δ; | 1045 cm⁻¹; | |
| | | 36.0 δ; | 1006 cm⁻¹; | |
| | | 28.8 δ; | 870 cm⁻¹; | |
| | | 26.2 δ; | 785 cm⁻¹; | |
| | | 23.7 δ; | 764 cm⁻¹ | |
| | | 22.9 δ | | |

| Table 2 | | | | |
|---|---|---|---|---|
| Dihydrotetrabenazine isomer | ¹H-NMR spectrum (CDCl₃) | ¹³C-NMR spectrum (CDCl₃) | IR Spectrum (KBr solid) | Mass Spectrum (ES⁺) |
| Isomers C and D | 6.68 δ 1H (s); | 147.8 δ; | 3370 cm⁻¹; | MH⁺ 320 |
| | 6.58 δ 1H (s); | 147.7 δ; | 2950 cm⁻¹; | |
| | 3.92 δ 1H (m); | 130.4 δ; | 2929 cm⁻¹; | |
| | 3.84 δ 6H (s); | 127.2 δ; | 1611 cm⁻¹; | |
| | 3.15 δ 1H (m); | 112.0 δ; | 1512 cm⁻¹; | |
| | 2.87 δ 3H (m); | 108.3 δ; | 1463 cm⁻¹ | |
| 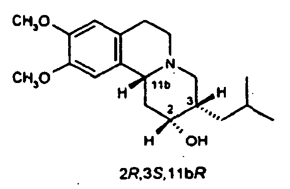 | 2.43 δ 4H (m); | 72.4 δ; | 1362 cm⁻¹; | |
| | 1.81 δ 1H (m); | 61.2 δ; | 1334 cm⁻¹; | |
| | 1.64 δ 4H (m); | 58.3 δ; | 1259 cm⁻¹; | |
| | 1.21 δ 1H (m); | 56.5 δ; | 1227 cm⁻¹; | |
| | 0.94 δ 3H (d); | 56.3 δ; | 1148 cm⁻¹; | |
| OR | 0.89 δ 3H (d) | 52.7 δ; | 1063 cm⁻¹; | |
| | | 38.6 δ; | 1024 cm⁻¹; | |
| 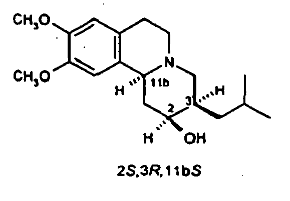 | | 36.7 δ; | 855 cm⁻¹ ; | |
| | | 34.4 δ; | 766 cm⁻¹ | |
| | | 29.6 δ; | | |
| | | 26.5 δ; | | |
| | | 24.4 δ; | | |
| | | 22.5 δ | | |

**Tables 3 and 4 - Chromatography and ORD Data**

| Table 3 | | | |
|---|---|---|---|
| Dihydrotetrabenazine isomer | Chiral HPLC Methods and Retention Times | | ORD (MeOH, 21°C) |
| Isomers A and B | Column: | | Isomer A |
| 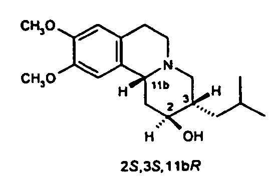 | Chirex (S)-VAL, (R)-NEA, 250 x 4.6 mm | | [α_{D}]-114.6° |
| | Mobile phase: Hexane : 1,2-dichloroethane : ethanol (36:62:2) | | |
| | Flow: | 1.0 ml min⁻¹ | Isomer B |
| | UV: | 254 nm | [α_{D}] +123° |
| OR | | | |
| 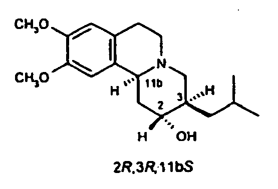 | Retention times: | | |
| | Isomer A | 16.6 min | |
| | Isomer B | 15.3 min | |

| Table 4 | | | |
|---|---|---|---|
| Isomers C and D | Column: | | Isomer C |
| 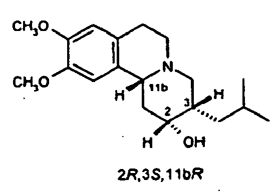 | Chirex (S)-VAL, (R)-NEA, 250 x 4.6mm | | [α_{D}] +150.9° |
| | Mobile phase: | Hexane : ethanol (92:8) | |
| | Flow: | 1.0 ml min⁻¹ | Isomer D |
| | UV: | 254 nm | [α_{D}] -145.7° |
| OR | Retention times: | | |
| 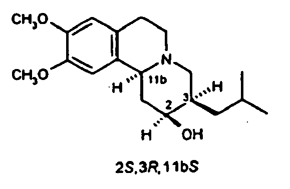 | Isomer C | 20.3 min | |
| | Isomer D | 19.4 min | |

### EXAMPLE 3

### Alternative Method of Preparation of Isomer B and Preparation of Mesylate Salt

### 3A. Reduction of RR/SS Tetrabenazine

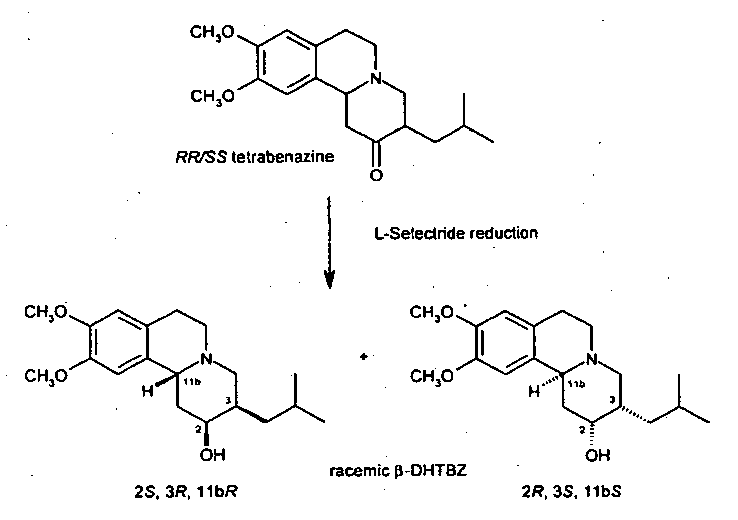

1M L-Selectride^{®} in tetrahydrofuran (52 ml, 52 mmol, 1.1 eq) was added slowly over 30 minutes to a cooled (ice bath), stirred solution of tetrabenazine racemate (15 g, 47 mmol) in tetrahydrofuran (56 ml). After the addition was complete, the mixture was allowed to warm to room temperature and stirred for a further six hours. TLC analysis (silica, ethyl acetate) showed only very minor amounts of starting material remained.

The mixture was poured on to a stirred mixture of crushed ice (112 g), water (56 ml) and glacial acetic acid (12.2 g). The resulting yellow solution was washed with ether (2 x 50 ml) and basified by the slow addition of solid sodium carbonate (ca. 13 g). Pet-ether (30-40 °C) (56 ml) was added to the mixture with stirring and the crude β-DHTBZ was collected as a white solid by filtration.

The crude solid was dissolved in dichloromethane (ca. 150 ml) and the resulting solution washed with water (40 ml), dried using anhydrous magnesium sulphate, filtered and concentrated at reduced pressure to ca. 40 ml. A thick suspension of white solid was formed. Pet-ether (30-40 °C) (56 ml) was added and the suspension was stirred for fifteen minutes at laboratory temperature. The product was collected by filtration and washed on the filter until snow-white using pet-ether (30-40°C) (40 to 60 ml) before air-drying at room temperature to yield β-DHTBZ (10.1 g, 67%) as a white solid. TLC analysis (silica, ethyl acetate) showed only one component.

### 3B Preparation and Fractional Crystallisation of the Camphorsulphonic acid Salt of Racemic β-DHTBZ

The product of Example 3A and 1 equivalent of (*S*)-(+)-Camphor-10-sulphonic acid were dissolved with heating in the minimum amount of methanol. The resulting solution was allowed to cool and then diluted slowly with ether until formation of the resulting solid precipitation was complete. The resulting white crystalline solid was collected by filtration and washed with ether before drying.

The camphorsulphonic acid salt of (10 g) was dissolved in a mixture of hot absolute ethanol (170 ml) and methanol (30 ml). The resulting solution was stirred and allowed to cool. After two hours the precipitate formed was collected by filtration as a white crystalline solid (2.9 g). A sample of the crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet-ether (30-40 °C) and the organic solution concentrated once more. Chiral HPLC analysis of the salt using a Chirex (S)-VAL and (R)-NEA 250 x 4.6 mm column, and a hexane : ethanol (98:2) eluent at a flow rate of 1 ml/minute showed showed that the isolated β-DHTBZ was enriched in one enantiomer (e.e. ca. 80%).

The enriched camphorsulphonic acid salt (14 g) was dissolved in hot absolute ethanol (140 ml) and propan-2-ol (420 ml) was added. The resulting solution was stirred and a precipitate began to form within one minute. The mixture was allowed to cool to room temperature and stirred for one hour. The precipitate formed was collected by filtration, washed with ether and dried to give a white crystalline solid (12 g).

The crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet-ether (30-40 °C) and the organic solution concentrated once more to yield (after drying in vacuo.) (+)-β-DHTBZ (6.6 g, ORD +107.8°). The isolated enantiomer has e.e. >97%.

### 3C. Preparation of Isomer B

A solution of phosphorus pentachloride (4.5 g, 21.6 mmol, 1.05 eq) in dichloromethane (55 ml) was added steadily over ten minutes to a stirred, cooled (ice-water bath) solution of the product of Example 3B (6.6 g, 20.6 mmol) in dichloromethane (90 ml). When the addition was complete, the resulting yellow solution was stirred for a further ten minutes before pouring on to a rapidly stirred mixture of sodium carbonate (15 g) in water (90 ml) and crushed ice (90 g). The mixture was stirred for a further 10 minutes and transferred to a separating funnel.

Once the phases had separated, the brown dichloromethane layer was removed, dried over anhydrous magnesium sulphate, filtered and concentrated at reduced pressure to give the crude alkene intermediate as brown oil (ca. 6.7 g). TLC analysis (silica, ethyl acetate) showed that no (+)-β-DHTBZ remained in the crude product.

The crude alkene was taken up (dry nitrogen atmosphere) in anhydrous tetrahydrofuran (40 ml) and a solution of borane in THF (1M solution, 2.5 eq, 52 ml) was added with stirring over fifteen minutes. The reaction mixture was then stirred at room temperature for two hours. TLC analysis (silica, ethyl acetate) showed that no alkene intermediate remained in the reaction mixture.

A solution of sodium hydroxide (3.7 g) in water (10 ml) was added to the stirring reaction mixture, followed by an aqueous solution of hydrogen peroxide (50%, ca. 7 ml) and the two-phase mixture formed was stirred at reflux for one hour. TLC analysis of the organic phase at this time (silica, ethyl acetate) showed the appearance of a product with Rf as expected for Isomer B. A characteristic nonpolar component was also seen.

The reaction mixture was allowed to cool to room temperature and was poured into a separating funnel. The upper organic layer was removed and concentrated under reduced pressure to remove the majority of THF. The residue was taken up in ether (stabilised (BHT), 75 ml), washed with water (40 ml), dried over anhydrous magnesium sulphate, filtered and concentrated under reduced pressure to give a pale yellow oil (8.1 g).

The yellow oil was purified using column chromatography (silica, ethyl acetate: hexane (80:20), increasing to 100% ethyl acetate) and the desired column fractions collected, combined and concentrated at reduced pressure to give a pale oil which was treated with ether (stabilised, 18 ml) and concentrated at reduced pressure to give Isomer B as a pale yellow solid foam (2.2 g).

Chiral HPLC using the conditions set out in Example 3B confirmed that Isomer B had been produced in an enantiomeric excess (e.e.) of greater than 97%.

The optical rotation was measured using a Bellingham Stanley ADP220 polarimeter and gave an [α_{D}] of +123.5°.

### 3D. Preparation of the Mesylate salt of Isomer B

The methanesulphonate salt of Isomer B was prepared by dissolving a mixture of 1 equivalent of Isomer B from Example 3C and 1 equivalent of methane sulphonic acid in the minimum amount of ethanol and then adding diethyl ether. The resulting white precipitate that formed was collected by filtration and dried *in vacuo* to give the mesylate salt in a yield of ca. 85% and a purity (by HPLC) of ca. 96%.

### EXAMPLE 4

### X-Ray Crystallographic Studies on Isomer B

The (*S*)-(+)-Camphor-10-sulphonic acid salt of Isomer B was prepared and a single crystal was subjected to X-ray crystallographic studies under the following conditions:
Diffractometer: Nonius KappaCCD area detector (t/i scans and OJ scans to fill asymmetric unit).
Cell determination: DirAx (Duisenberg, A.J.M.(1992). J. Appl. Cryst. 25, 92-96.)
Data collection: Collect (Collect: Data collection software, R. Hooft, Nonius B. V, 1998)
Data reduction and cell refinement: Demo (Z. Otwinowski & W. Minor, Methods in Enzymology (1997) Vol. 276: Macromolecular Crystallography, part A, pp. 307-326; C. W. Carter, Jr & R. M. Sweet, Eds., Academic Press).
Absorption correction: Sheldrick, G. M. SADABS - Bruker Nonius area detector scaling and absorption correction - V2.\ 0
Structure solution: SHELXS97 (G. M. Sheldrick, Acta Cryst. (1990) A46 467-473). Structure refinement: SHELXL97 (G. M. Sheldrick (1997), University of Göttingen, Germany)
Graphics: Cameron - A Molecular Graphics Package (D. M. Watkin, L. Pearce and C. K. Prout, Chemical Crystallography Laboratory, University of Oxford, 1993)
Special details: All hydrogen atoms were placed in idealised positions and refined using a riding model, except those of the NH and OH which were located in the difference map and refined using restraints. Chirality: NI=R, CI2=S, CI3=S, CI5=R, C21=S, C24=R

The results of the studies are set out below in Tables A, B, C, D and E.

In the Tables, the label RUS0350 refers to Isomer B.

**TABLE A**

| | | |
|---|---|---|
| Identification code | **2005bdy0585 (RUS0350)** | |
| Empirical formula | C₂₉H₄₅NO₇S | |
| Formula weight | 551.72 | 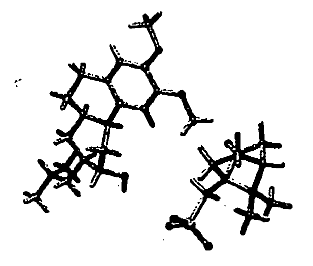 |
| Temperature | 120(2) K | |
| Wavelength | 0.71073 Å | |
| Crystal system | Orthorhombic | |
| Space group | *P*2₁2₁2₁ | |
| Unit cell dimensions | *a* = 7.1732(9) Å | |
| | *b* = 12.941(2) Å | |
| | *c* = 31.025(4)Å | |
| Volume | 2880.1(7) Å³ | |
| Z | 4 | |
| Density (calculated) | 1.272 Mg/m³ | |
| Absorption coefficient | 0.158 mm⁻¹ | |
| *F(000)* | 1192 | |
| Crystal | Colourless Slab | |
| Crystal size | 0.2 x 0.2 x 0.04 mm³ | |
| θ range for data collection | 3.06 - 27.37° | |
| Index ranges | -8 ≤ *h* ≤ 9, -16 ≤ *k* ≤ 16, -36 ≤ *l* ≤ 39 | |
| Reflections collected | 36802 | |
| Independent reflections | 6326 [*Rᵢₙₜ* = 0.0863] | |
| Completeness to θ= 27.37° | 97.1 % | |
| Absorption correction | Semi-empirical from equivalents | |
| Max. and min. transmission | 0.9937 and 0.9690 | |
| Refinement method | Full-matrix least-squares on *F*² | |
| Data / restraints / parameters | 6326 / 1 / 357 | |
| Goodness-of-fit on *F*² | 1.042 | |
| Final *R* indices [*F*² > 2σ(*F*²)] | *R1* = 0.0498, *wR2*= 0.0967 | |
| *R* indices (all data) | *R1* = 0.0901, *wR2* = 0.1108 | |
| Absolute structure parameter | 0.04(8) | |
| Extinction coefficient | 0.0059(7) | |
| Largest diff. peak and hole | 0.236 and -0.336 e Å⁻³ | |

**TABLE B. Atomic coordinates [x 10⁴], equivalent isotropic displacement parameters [A² x 10³] and site occupancy factors. U_{eq} is defined as one third of the trace of the orthogonalized U^{ij} tensor.**

| Atom | *x* | *y* | *z* | *Ueq* | *S*.*o*.*f*. |
|---|---|---|---|---|---|
| Nl | 4839(3) | 111119(2) | 2180(1) | 24(1) | 1 |
| 01 | 2515(3) | 13171(1) | 349(1) | 31(1) | 1 |
| 02 | 5581(3) | 14030(1) | 598(1) | 32(1) | 1 |
| 03 | 9220(3) | 12834(2) | 2385(1) | 36(1) | 1 |
| Cl | 870(4) | 12674(2) | 190(1) | 36(1) | 1 |
| C2 | 3176(3) | 12838(2) | 739(1) | 25(1) | 1 |
| C3 | 2346(4) | 12109(2) | 997(1) | 25(1) | 1 |
| C4 | 3124(3) | 11821(2) | 1395(1) | 24(1) | 1 |
| C5 | 4773(3) | 12276(2) | 1527(1) | 23(1) | 1 |
| C6 | 5629(4) | 13024(2) | 1262(1) | 24(1) | 1 |
| C7 | 4861(4) | 13308(2) | 875(1) | 25(1) | 1 |
| C8 | 7189(4) | 14582(2) | 747(1) | 38(1) | 1 |
| C9 | 2182(3) | 11023(2) | 1673(1) | 28(1) | 1 |
| C10 | 2759(3) | 11118(2) | 2137(1) | 26(1) | 1 |
| C11 | 5366(3) | 11096(2) | 2656(1) | 25(1) | 1 |
| C12 | 7292(4) | 11536(2) | 2747(1) | 25(1) | 1 |
| C13 | 7468(4) | 12663(2) | 2590(1) | 25(1) | 1 |
| C14 | 5988(4) | 12911(2) | 2252(1) | 25(1) | 1 |
| C15 | 5773(4) | 12010(2) | 1943(1) | 24(1) | 1 |
| C16 | 7734(4) | 11477(2) | 3232(1) | 28(1) | 1 |
| C17 | 7752(4) | 10418(2) | 3449(1) | 34(1) | 1 |
| C18 | 9198(6) | 9696(3) | 3249(1) | 65(1) | 1 |
| C19 | 8114(4) | 10562(2) | 3930(1) | 41(1) | 1 |
| C20 | 7509(4) | 8131 (2) | 1250(1) | 31(1) | 1 |
| S1 | 7409(1) | 8792(1) | 1754(1) | 27(1) | 1 |
| 04 | 7758(2) | 7965(1) | 2064(1) | 30(1) | 1 |
| 05 | 8831 (3) | 9582(2) | 1760(1) | 49(1) | 1 |
| 06 | 5524(2) | 9221(1) | 1798(1) | 32(1) | 1 |
| 07 | 7406(3) | 6932(1) | 498(1) | 48(1) | 1 |
| C21 | 6858(3) | 8622(2) | 830(1) | 25(1) | 1 |
| C22 | 7154(4) | 7851 (2) | 459(1) | 30(1) | 1 |
| C23 | 7073(4) | 8450(2) | 40(1) | 32(1) | 1 |
| C24 | 6648(3) | 9544(2) | 203(1) | 28(1) | 1 |
| C25 | 4742(3) | 8877(2) | 787(1) | 29(1) | 1 |
| C26 | 4742(3) | 8877(2) | 787(1) | 29(1) | 1 |
| C27 | 7773(4) | 9610(2) | 630(1) | 25(1) | 1 |
| C28 | 7431(4) | 10628(2) | 868(1) | 29(1) | 1 |
| C29 | 9895(4) | 9489(2) | 569(1) | 36(1) | 1 |

**TABLE C. Bond lengths [A] and angles [°].**

| | | | |
|---|---|---|---|
| Nl-ClO | 1.498(3) | C14-C15 | 1.518(3) |
| Nl-Cl5 | 1.522(3) | C16-C17 | 1.526(3) |
| Nl-Cll | 1.524(3) | C17-C18 | 1.527(4) |
| 01-C2 | 1.368(3) | C17-C19 | 1.527(4) |
| Ol-Cl | 1.432(3) | C20-C21 | 1.525(3) |
| 02-C7 | 1.369(3) | C20-Sl | 1.784(2) |
| 02-C8 | 1.433(3) | Sl-05 | 1.4442(19) |
| 03-C13 | 1.425(3) | Sl-04 | 1.4607(17) |
| C2-C3 | 1.372(3) | Sl-06 | 1.4676(18) |
| C2-C7 | 1.417(3) | 07-C22 | 1.208(3) |
| C3-C4 | 1.407(3) | C21-C22 | 1.537(4) |
| C4-C5 | 1.384(3) | C21-C26 | 1.559(3) |
| C4-C9 | 1.506(3) | C21-C27 | 1.565(3) |
| C5-C6 | 1.411(3) | C22-C23 | 1.517(4) |
| C5-C15 | 1.516(3) | C23-C24 | 1.535(4) |
| C6-C7 | 1.372(3) | C24-C25 | 1.548(4) |
| C9-C10 | 1.504(3) | C24-C27 | 1.554(4) |
| Cl-Cl2 | 1.521 (3) | C25-C26 | 1.557(4) |
| C12-C16 | 1.540(3) | C27-C28 | 1.529(3) |
| C12-C13 | 1.544(3) | C27-C29 | 1.542(4) |
| C13-C14 | 1.524(3) | | |
| | | | |
| Cl0-Nl-Cl5 | 113.33(19) | Cl2-Cll-Nl | 113.43(19) |
| Cl0-Nl-Cll | 109.46(18) | Cll-Cl2-Cl6 | 110.5(2) |
| Cl5-Nl-Cll | 111.96(19) | Cll-Cl2-Cl3 | 111.7(2) |
| C2-01-Cl | 116.6(2) | Cl6-Cl2-Cl3 | 109.84(19) |
| C7-02-C8 | 116.27(19) | 03-Cl3-Cl4 | 106.0(2) |
| 01-C2-C3 | 125.5(2) | 03-Cl3-C12 | 111.1 (2) |
| 01-C2-C7 | 115.0(2) | Cl4-Cl3-Cl2 | 111.0(2) |
| C3-C2-C7 | 119.5(2) | Cl5-Cl4-Cl3 | 110.1 (2) |
| C2-C3-C4 | 121.5(2) | C5-C15-C14 | 114.3(2) |
| C5-C4-C3 | 119.2(2) | C5-Cl5-Nl | 112.0(2) |
| C5-C4-C9 | 120.3(2) | Cl4-Cl5-Nl | 108.7(2) |
| C3-C4-C9 | 120.5(2) | Cl7-Cl6-Cl2 | 118.4(2) |
| C4-C5-C6 | 119.4(2) | Cl6-Cl7-Cl8 | 112.2(2) |
| C4-C5-Cl5 | 124.1(2) | Cl6-Cl7-Cl9 | 108.7(2) |
| C6-C5-Cl5 | 116.6(2) | Cl8-Cl7-Cl9 | 110.8(3) |
| C7-C6-C5 | 121.3(2) | C21-C20-S1 | 122.51(18) |
| 02-C7-C6 | 125.4(2) | 05-Sl-04 | 112.93(11) |
| 02-C7-C2 | 115.4(2) | 05-Sl06 | 112.47(12) |
| C6-C7-C2 | 119.2(2) | 04-Sl-06 | 111.93(11) |
| Cl0-C9-C4 | 111.7(2) | 05-Sl-C20 | 108.81 (13) |
| Nl-Cl0-C9 | 111.0(2) | 04-Sl-C20 | 102.60(11) |
| | | | |
| 06-Sl-C20 | 107.44(12) | C23-C24-C25 | 106.4(2) |
| C20-C21-C22 | 109.0(2) | C23-C24-C27 | 103.3(2) |
| C20-C21-C26 | 117.3(2) | C25-C24-C27 | 102.3(2) |
| C22-C21-C26 | 102.1 (2) | C24-C25-C26 | 102.9(2) |
| C20-C21-C27 | 123.4(2) | C25-C26-C21 | 104.2(2) |
| C22-C21-C27 | 100.21(19) | C28-C27-C29 | 107.8(2) |
| C26-C21-C27 | 101.7(2) | C28-C27-C24 | 112.0(2) |
| 07-C22-C23 | 126.4(2) | C29-C27-C24 | 113.7(2) |
| 07-C22-C21 | 125.9(2) | C28-C27-C21 | 116.5(2) |
| C23-C22-C21 | 107.7(2) | C29-C27-C21 | 112.3(2) |
| C22-C23-C24 | 101.3(2) | C24-C27-C21 | 94.27(19) |

**TABLE D. Anisotropic displacement parameters [A² x 10³]. The anisotropic displacement factor exponent takes the form:- 2π²[h²a*²U¹¹ + ... + 2 h k a* b* U¹²].**

| Atom | *U¹¹* | *U²²* | *U³³* | *U²³* | *U¹³* | *U¹²* |
|---|---|---|---|---|---|---|
| Nl | 26(1) | 24(1) | 23(1) | 2(1) | -1(1) | -3(1) |
| 01 | 37(1) | 30(1) | 24(1) | 3(1) | -7(1) | -4(1) |
| 02 | 41(1) | 31(1) | 25(1) | 5(1) | -2(1) | -10(1) |
| 03 | 26(1) | 49(1) | 32(1) | 7(1) | -3(1) | -9(1) |
| Cl | 41(2) | 36(2) | 32(2) | 3(1) | -9(1) | -8(2) |
| C2 | 30(2) | 24(2) | 22(1) | 1 (1) | -1(1) | 2(1) |
| C3 | 25(1) | 26(1) | 24(1) | -3(1) | -2(1) | 2(1) |
| C4 | 26(2) | 22(1) | 23(1) | -1(1) | 2(1) | -1(1) |
| C5 | 24(1) | 22(1) | 23(1) | -2(1) | 1(1) | 0(1) |
| C6 | 26(1) | 22(1) | 24(1) | -3(1) | 2(1) | -5(1) |
| C7 | 30(2) | 22(1) | 22(1) | 2(1) | 4(1) | -4(1) |
| C8 | 45(2) | 34(2) | 36(2) | 5(1) | -2(1) | -20(2) |
| C9 | 23(1) | 32(1) | 29(2) | 3(1) | -1(1) | -4(1) |
| ClO | 26(1) | 29(1) | 25(1) | 2(1) | 0(1) | -5(1) |
| C11 | 31(1) | 25(1) | 20(1) | 2(1) | 0(1) | -2(1) |
| C12 | 26(1) | 26(1) | 23(1) | -1(1) | 1(1) | -1(1) |
| Cl3 | 26(1) | 28(1) | 23(1) | -1(1) | -1(1) | -2(1) |
| Cl4 | 30(2) | 22(2) | 24(1) | -1(1) | 1(1) | -1(1) |
| Cl5 | 22(1) | 22(1) | 28(1) | 2(1) | 0(1) | -4(1) |
| C16 | 31(1) | 28(1) | 24(1) | -1(1) | -3(1) | 3(1) |
| Cl7 | 46(2) | 31 (2) | 25(1) | 1(1) | -7(1) | 0(2) |
| Cl8 | 106(3) | 46(2) | 41 (2) | 6(2) | -1 (2) | 31 (2) |
| C19 | 51(2) | 41 (2) | 31 (2) | 9(2) | -7(1) | -4(2) |
| C20 | 30(2) | 34(2) | 29(1) | 2(1) | 3(1) | 9(2) |
| S1 | 27(1) | 30(1) | 24(1) | 4(1) | -2(1) | -5(1) |
| 04 | 31(1) | 36(1) | 23(1) | 9(1) | -1(1) | 0(1) |
| 05 | 53(1) | 58(1) | 37(1) | 13(1) | -11(1) | -35(1) |
| 06 | 34(1) | 35(1) | 28(1) | -3(1) | -2(1) | 10(1) |
| 07 | 81(2) | 25(1) | 40(1) | -1(1) | 12(1) | 6(1) |
| C21 | 26(1) | 25(2) | 24(1) | -1 (1) | 3(1) | 2(1) |
| C22 | 35(2) | 25(2) | 31(2) | 0(1) | 1 (1) | -1(1) |
| C23 | 40(2) | 30(2) | 25(1) | -2(1) | 1 (1) | -2(1) |
| C24 | 28(1) | 29(2) | 26(2) | 2(1) | 2(1) | 2(1) |
| C25 | 30(2) | 34(2) | 29(2) | -1 (1) | -2(1) | 0(1) |
| C26 | 26(1) | 34(2) | 28(2) | 0(1) | 1(1) | -5(1) |
| C27 | 23(1) | 26(1) | 26(1) | 0(1) | 2(1) | 0(1) |
| C28 | 31(1) | 26(1) | 30(1) | 0(1) | -2(1) | -6(1) |
| C29 | 29(2) | 41 (2) | 40(2) | 0(2) | 2(1) | -3(1) |

**TABLE E. Hydrogen coordinates [x 10⁴] and isotropic displacement parameters [Å² x 10³].**

| Atom | *x* | *y* | *z* | *U_{eq}* | *S*.*o*.*f* |
|---|---|---|---|---|---|
| H98 | 5190(40) | 10528(15) | 2062(10) | 70(8) | 1 |
| H99 | 10030(50) | 12950(30) | 2575(12) | 70(8) | 1 |
| H1A | 1107 | 11933 | 156 | 54 | 1 |
| H1B | 529 | 12973 | -89 | 54 | 1 |
| H1C | -154 | 12777 | 395 | 54 | 1 |
| H3 | 1220 | 11793 | 904 | 30 | 1 |
| H6 | 6760 | 13337 | 1353 | 29 | |
| H8A | 6872 | 14966 | 1009 | 58 | 1 |
| H8B | 7600 | 15065 | 523 | 58 | 1 |
| H8C | 8193 | 14091 | 810 | 58 | 1 |
| H9A | 814 | 11106 | 1651 | 33 | 1 |
| H9B | 2505 | 10324 | 1567 | 33 | 1 |
| H10A | 2250 | 11767 | 2259 | 32 | 1 |
| H10B | 2235 | 10534 | 2304 | 32 | 1 |
| H11A | 4431 | 11494 | 2822 | 30 | 1 |
| H11B | 5322 | 10372 | 2759 | 30 | 1 |
| H12 | 8230 | 11108 | 2589 | 30 | 1 |
| H13 | 7334 | 13145 | 2840 | 30 | 1 |
| H14A | 4783 | 13050 | 2397 | 30 | 1 |
| H14B | 6354 | 13538 | 2090 | 30 | 1 |
| H15 | 7056 | 11776 | 1864 | 29 | 1 |
| H16A | 8973 | 11796 | 3278 | 33 | 1 |
| H16B | 6813 | 11911 | 3386 | 33 | 1 |
| I H17 | 6493 | 10098 | 3412 | 41 | 1 |
| H18A | 8906 | 9588 | 2944 | 97 | 1 |
| H18B | 9176 | 9031 | 3400 | 97 | 1 |
| H18C | 10440 | 10005 | 3276 | 97 | 1 |
| H19A | 9329 | 10894 | 3971 | 62 | 1 |
| H19B | 8110 | 9887 | 4073 | 62 | 1 |
| H19C | 7135 | 10999 | 4054 | 62 | 1 |
| H20A | 8824 | 7924 | 1207 | 37 | 1 |
| H20B | 6787 | 7484 | 1286 | 37 | 1 |
| H23A | 6070 | 8190 | -151 | 38 | 1 |
| H23B | 8277 | 8423 | -116 | 38 | 1 |
| H24 | 6928 | 10107 | -8 | 33 | 1 |
| H25A | 3773 | 9195 | 153 | 37 | 1 |
| H25B | 4152 | 10235 | 426 | 37 | 1 |
| H26A | 3994 | 8237 | 764 | 35 | 1 |
| H26B | 4300 | 9279 | 1039 | 35 | 1 |
| H28A | 8160 | 10638 | 1135 | 44 | 1 |
| I H28B | 6103 | 10692 | 936 | 44 | 1 |
| H28C | 7811 | 11207 | 684 | 44 | 1 |
| H29A | 10358 | 10042 | 381 | 54 | 1 |
| H29B | 10159 | 8817 | 436 | 54 | 1 |
| H29C | 10517 | 9531 | 849 | 54 | 1 |

**Table 6. Hydrogen bonds [Å and °].**

| *D*-H*···A* | *d*(*D*-H) | *d*(H···*A*) | *d*(*D···A*) | ∠(*D*H*A*) |
|---|---|---|---|---|
| N1-H98···O6 | 0.885(10) | 1.895(12) | 2.773(3) | 171(3) |
| N1-H98···S1 | 0.885(10) | 2.914(14) | 3.771(2) | 163(3) |
| O3-H99···O4ⁱ | 0.84(4) | 1.94(4) | 2.766(3) | 165(3) |
| O3-H99···S1ⁱ | 0.84(4) | 2.98(4) | 3.811(2) | 169(3) |
| Symmetry transformations used to generate equivalent atoms: | | | | |
| (i) -x+2,y+ 1/2,-z+ 1/2 | | | | |

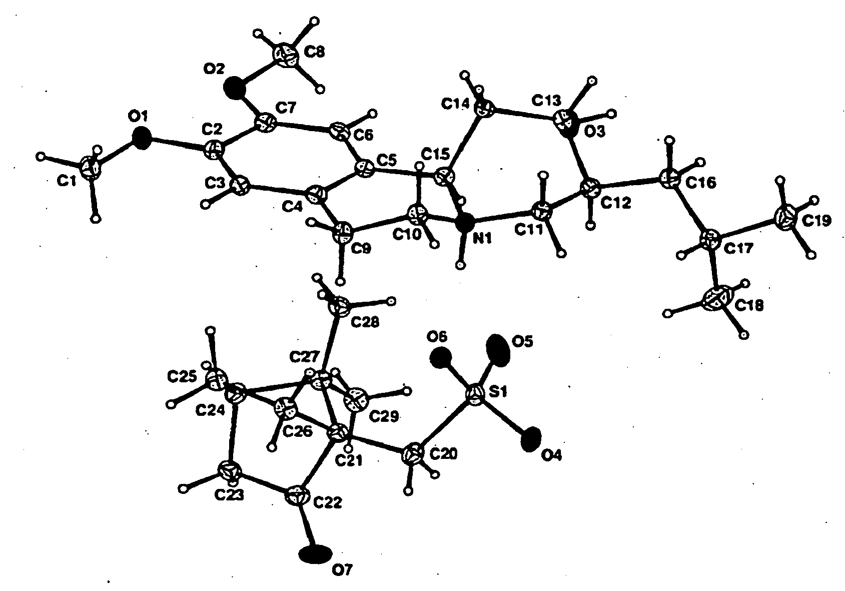

**Thermal ellipsoids drawn at the 30% probability level**

On the basis of the data set out above, Isomer B is believed to have the 2S, 3S, 11bR configuration, which corresponds to Formula (Ia):

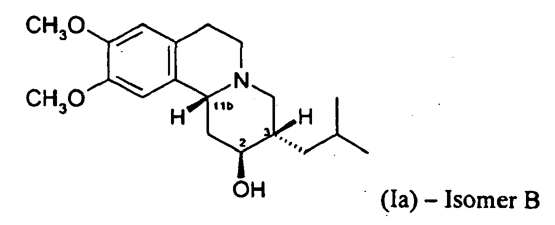

### EXAMPLE 5

### Sigma Receptor Binding Studies

The four dihydrotetrabenazine isomers A, B, C and D were subjected to specific binding assays to test their ability to bind to the sigma-1 and sigma-2 receptors. The results are set out in Table 5.
**(a) Sigma** σ**₁ Receptor:**

| | |
|---|---|
| Reference: | Ganapathy et al., Pharmacol. Exp. Ther., 289:251-260, (1999) |
| Source: | Human jurkat cells |
| Ligand: | 8 nM [3H] Haloperidol |
| Vehicle: | 1% DMSO |
| Incubation time/Temp: | 4 hours @ 25 °C |
| Incubation buffer: | 5 mM K2HPO4/KH2PO4 buffer pH 7.5 |
| Non Specific ligand: | 10 µM Haloperidol |
| K_{d}: | 5.8 nM |
| Bₘₐₓ: | 0.71 pmole/mg protein |
| Specific binding: | 80% |
| Quantitation method: | Radioligand binding |
| Significance criteria: | ≥50% of maximum stimulation or inhibition |

**(b) Sigma** σ**₂ Receptor:**

| | |
|---|---|
| Reference: | Hashimoto et al., Eur. J. Pharmacol., 236:159-163, (1993) |
| Source: | Wistar rat brain |
| Ligand: | 3 nM [3H] Ifenprodil |
| Vehicle: | 1% DMSO |
| Incubation time/Temp: | 60 minutes @ 37°C |
| Incubation buffer: | 50 mM Tris-HCl, pH 7.4 |
| Non Specific ligand: | 10 µM Ifenprodil |
| K_{d}: | 4.8 nM |
| Bₘₐₓ: | 1.3 pmole/mg protein |
| Specific binding: | 85% |
| Quantitation method: | Radioligand binding |
| Significance criteria: | ≥ 50% of maximum stimulation or inhibition |

**Table 5**

| Percentage Inhibition by 10 µM Solutions of Dihydrotetrabenazine isomers of Specific Binding at Sigma-1 and Sigma-2 Receptors | | | | |
|---|---|---|---|---|
| Receptor/Protein | Isomer A | Isomer B | Isomer C | Isomer D |
| (g) σ₁ Receptor | 48 | 82 | 59 | 82 |
| (h) σ₂ Receptor | 64 | 64 | 61 | 69 |

The data show that all four isomers bind to both the sigma-1 and sigma-2 receptors. The four isomers are approximately equipotent in the sigma-2 receptor binding studies but isomers B and D show stronger binding to the sigma-1 receptor.

### EXAMPLE 6

### Determination of the effect of compounds of the invention on LPS/IFN stimulated production of cytokines by human monocytes

The compounds of the invention were tested to determine the extent to they can modulate the production of cytokines by human monocytes. Monocytes are precursors of macrophages and are a key source of inflammatory cytokines in a wide range of disease states. The activity of the compounds of the invention against monocyte production therefore provides a good indicator of the likelihood of the compounds possessing anti-inflammatory activity.

The following treatment groups were set up.

| **Group** | **Treatment** |
|---|---|
| A | Instimulated Monocyte Control |
| F | Instimulated Monocytes+Nabilone 100µM/10µM/1µM/100nM/10nM/1nM/100pM/10pM |
| G | Instimulated Monocytes + Isomer D (100µM/10µM/1µM/100nM/10nM/1nM/100pM/10pM |
| H | Instimulated Monocytes + Isomer B (100µM/10µM/1µM/100nM/10nM/1nM/100pM/10pM |
| I | LPS/IFNγ Stimulated Monocytes alone |
| N | Stimulated Monocytes + Nabilone (100µM/10µM/1µM/100nM/10nM/1nM/100pM/10pM |
| O | Stimulated Monocytes + Isomer D (100µM/10µM/1µM/100nM/10nM/1nM/100pM/10pM) |
| P | Stimulated Monocytes + Isomer B (100µM/10µM/1µM/100nM/10nM/1nM/100pM/10pM) |

### Procedures

### Modulation of human monocyte cytokine production

### Materials

MACS Buffer: 1 x PBS pH 7.2, 2 mM EDTA (Sigma), 5% human AB serum
Culture Media: 500 ml RPMI 1640 (Invitrogen), 5 ml Penstrep (Sigma), 5 ml L-glutamine (Invitrogen), 10 ml HEPES (Sigma), 5% autologous plasma.
Compounds: Stock at 10 mM in absolute ethanol.
LPS: Salmonella abortis at 1mg/ml (Sigma, Cat # L1887).
IFNγ: Recombinant human at 10 µg/ml (BD Pharmacia, Cat # 554617).
MACS CD14-positive selection beads and LS columns (Miltenyi Biotech).
CD14:FITC antibody (Miltenyi Biotech)
Buffy coat obtained from Bristol National Blood Services.
1 ml of blood should give 7 x 10⁶ total cells.

### Preparation of Autologous Plasma

Spin buffycoat at 3500 rpm, 10 min.
Remove upper plasma layer and heat-inactivate at 56 °C, 30 min.
Spin at 3500 rpm, 10 min to remove heat-inactivated complement proteins etc.

### Peripheral Blood Mononuclear Cell (PBMC) Isolation

1. Resuspend remaining buffycoat in an equal volume of RPMI 1640 medium and mix by inversion.
2. Prewarm histopaque (Sigma) to room temperature (RT) and add 10 ml to a universal.
3. Gently overlay with 15 ml blood/medium mix.
4. Spin at 1600 rpm for 25 minutes at RT (no brake).
5. PBMC are separated into the interface layer between the medium and the histopaque, remove cells to a 50 ml Falcon and add 10 ml medium.
6. Spin at 1800 rpm for 10 min at RT.
7. Wash x3 with 40 ml RPMI medium and resuspend in culture medium.

### Isolate CD14⁺ cells by positive selection with CD14⁺ MicroBeads

Work on ice, with pre-cooled solutions.
1. Determine cell numbers (313 x 10 x 5 x 10⁴ =1.565 x 10⁸ total cells)
2. Remove 100 µl cell sample for Fluorescence-Activated Cell-Sorting (FACS) analysis (pre-selection sample).
3. Spin at 1800 rpm for 10 minutes at 4 °C. Remove supernatant.
4. Resuspend pellet in 80 µl buffer per 1 x 10⁷ total cells (2400 µl/total cells).
5. Add 20 µl CD 14 MicroBeads per 1 x 10⁷ total cells (400 µl/total cells).
6. Mix well and incubate for 15 minutes at 4-8 °C.
7. Wash cells with 1-2 ml buffer, spin at 300 x g, 10 minutes at 4 °C. Remove supernatant.
8. Resuspend up to 1 x 10⁸ cells in 500 µl buffer (1 ml/total cells).

### Magnetic Separation with LS Column

1. Place column in magnetic field of MACS^{®} Separator. Rinse column with 3 ml of MACS buffer.
2. Add cell suspension to column.
3. Collect unlabelled cells which pass through. Wash column with 3x 3 ml buffer, allowing the column reservoir to empty between each wash. Collect total effluent (unlabelled cell fraction).
4. Remove column from separator and place on collection tube.
5. Add 5 ml buffer to column, immediately flushing out fraction with magnetically labelled cells by applying column plunger.
6. Spin cells in MACS Buffer and resuspend cell pellet in media.
7. Perform cell count on positively selected cells.
   71 x 20 x 5 x 10⁴ = 7.1 x 10⁷ cells/ml. Have 1.75ml □1.24 x 10⁸ total cells.
8. Take sample to check purity by FACS - add 10 µl CD14-FITC antibody and incubate, 5 minutes at 4-8 °C.

### Compound Preparation

Prepare compounds at 10 mM in absolute ethanol. Dilute prior to culture in media to the required concentrations.

### Cell Culture

Isolated CD14⁺ monocytes were cultured in 24-well plates at 1 x 10⁶ cells/ml/well. The monocytes were cultured in the presence of the test compounds and controls at various dilutions as set out in the table below. After overnight incubation, LPS (10 µg/ml) and IFNγ (100 ng/ml) were added to stimulate monocyte activation and elevate CB2 expression. After 48 hours, the supernatants were removed for CBA cytokine analysis.

### CBA Cytokine Analysis (BenderMedSystems Human Th1/Th2 Kit [Cat # BMS716FF])

Bring all buffers to room temperature and vortex before use
1. Make up assay buffer by adding to 450 ml distilled H₂O and mixing gently. Store at 4 °C.
2. Make up biotin-conjugate mix by 600 µl of each biotin-conjugates into a universal.
3. Make up bead mix by adding 300 µl of each bead set.
4. Reconstitute each standard with distilled H₂O as recommended on vial label. Add 100 µl assay buffer to tube labelled Standard 1. Add 10 µl of each reconstituted standard and mix. Serial dilute standard mixture (100 µl assay buffer to tubes 2-7, add 50 µl standard 1 to tube 2, mix and transfer 50 µl to tube 3 etc).
5. Label FACS tubes and add 25 µl of standards 1-7 to designated tubes. Add 25 µl assay buffer to blank tube.
6. Add 25 µl of sample to designated sample tubes.
7. Add 25 µl bead mix to all tubes.
8. Add 50 µl biotin-conjugate to all tubes.
9. Mix by vortexing, then cover with foil and incubate for 2 hours @ RT.
10. Prepare Strepavidin-PE solution by mixing 176 µl in 5324 µl assay buffer. Add 1 ml assay buffer to all tubes and spin at 200 x g, 5 min. Tip off supernatant. Repeat wash step and tip off supernatant.
11. Add 50 µl Strepavidin-PE to all tubes. Mix by vortexing, then cover with foil and incubate for 1h @ RT.
12. Repeat wash step x2.
13. Add 500 µl assay buffer to all tubes.

### Flow Cytometer Set-up

Run positive and negative beads (supplied with kit) to optimise set-up and determine compensation etc. Run standard curve prior to samples, counting 3000 events on gated population (300 events/analyte).

### RESULTS

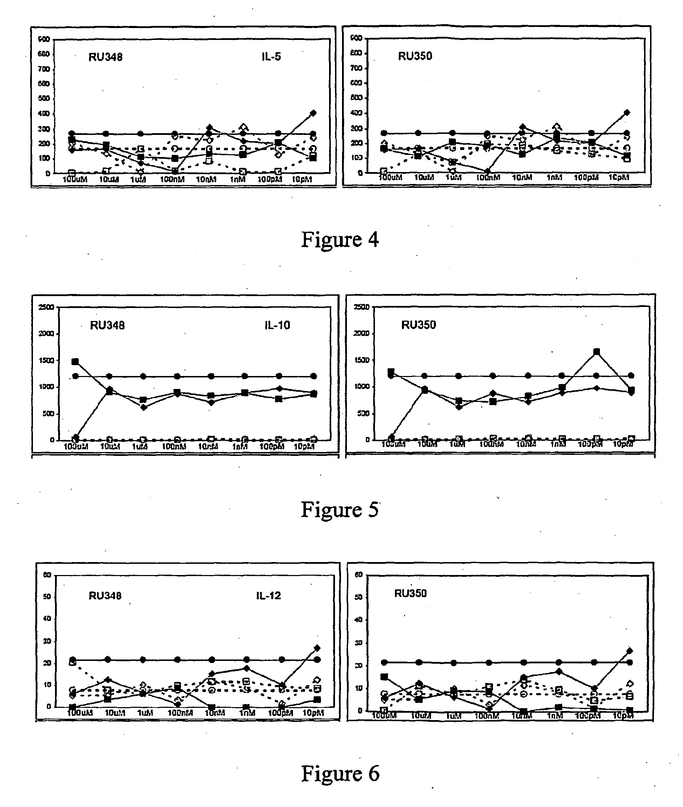

The results are shown in Figures 1 to 6. In the Figures, the label RU348 designates Isomer D.

As can be seen from Figures 1 to 6, the two cis-dihydrotetrabenazine isomers B and D both reduced the production of a range of different cytokines in monocytes that had been stimulated by LPS/IFN.

Thus, Isomer B reduced production of the cytokines IL-2 and IL-12 at all concentrations tested and reduced production of cytokines IL-4, IL-5 and IL-10 at the majority of concentrations tested.

Isomer D reduced production of the cytokines IL-2, IL-4, IL-5 and IL-12 at all concentrations tested and reduced production of cytokines TNFa, IL-4, IL-5 and IL-10 at the majority of concentrations tested.

### EXAMPLE 7

### Determination of the effect of compounds of the invention on T Cell Proliferation

The compounds of the invention were tested to determine the extent to they can inhibit T cell proliferation. T cells are responsible for co-ordinating the adaptive immune response and are drivers of inflammation in a wide range of disease states. The activity of the compounds of the invention against T cell proliferation therefore provides a good indicator of the likelihood of the compounds possessing anti-inflammatory activity.

### Materials

HBSS + 2% HEPES buffer (500 ml+10 ml), Alpha MEM(500 ml) + 2% HEPES(10 ml) + 1% Pen/strep(5 ml) + 2% L-Glutamine(10ml) + 50µM 2-ME(500 µl) and Normal Mouse serum (NMS)

### Methods

1. Coat 3 x 96 well plates columns 1-8, rows A-H with 100µl anti-mouse CD3 at 1 µg/ml in PBS. 192 well in total, therefore made up 24 mls in PBS by adding 17 µl of stock at 1.44mg/ml. Incubate 2 hours 37 °C.
2. Pooled blood from 2 balb/c mice following cardiac puncture for preparation of NMS and removed spleens into HBSS + Hepes.
3. Placed blood at 37° C for 30 minutes to clot, spun 3,000 rpm, 10 minutes in chilspin. Removed serum, filter sterilised and kept in fridge until use.
4. Transferred spleens to petri dishes containing 10 ml HBSS, used clean sterile wire mesh placed over spleen to release cells using a glass rod. Washed cells off mesh using pasteur pipette and transferred to a 15ml tube. Spun at 1000 rpm for 10 minutes at room temperature (RT).
5. Resuspended cells, added 0.5ml sterile DW to lyse RBC, mixed and immediately diluted in HBSS, transfered to 50 ml tube passing through cell strainer (70µM) to remove debris, spun as above, washed cells once more in HBSS and finally resuspended in 2mls alpha MEM containing supplements (as above).
6. Cell counts - 252 cells x 2 x 5 x 10⁴ = 2.52 x 10⁷ cells/ml in 4mls = 10⁸ cells in total. Dilute 2mls to 25mls to give a cell concentration of 2 x 10⁶ cells/ml.
7. Added 250µl NMS to cells to give a concentration of 1 %.
8. Washed anti-CD3 coated plates 3 x PBS.
9. Transferred 100µl aliquots of cells to coated wells, rows A-H columns 1-8 of 3 x 96-well plates. Add equal volume of compounds diluted in a 96 well plate as shown below.

### Compounds

Transferred 3.5 µl compound at 10 mM to 346.5µl media (1/100), transferred 35 µl of 1/100 dilution to 315 µl media (1/10) and so on down plate to give serial 1/10 dilutions of compound. Transferred 100µl aliquots of different compound dilutions to wells of triplicate plates containing T cells.

| **Nab*** | **Isomer D** | **Isomer B** | **Media** |
|---|---|---|---|
| 1/100 | 100µM | 100µM | 100µM |
| 1/10 | 10µM | 10µM | 10µM |
| 1/10 | 1µM | 1µM | 1µM |
| 1/10 | 100nM | 100nM | 100nM |
| 1/10 | 10nM | 10nM | 10nM |
| 1/10 | 1nM | 1nM | 1nM |
| 1/10 | 100pM | 100pM | 100pM |
| 1/10 | 10pM | 10pM | 10pM |

| | | | |
|---|---|---|---|
| * Nan = nabilone | | | |

Culture for 48 hours, then add a drop (16µl) of ³H-thymidine to each well and incubate overnight at 37° C 5% CO₂. Harvest. Measure ³H-thymidine incorporation.

### Results

The extent of T call proliferation, as demonstrated by levels of ³H-thymidine incorporation, is demonstrated by the data are shown in the Table below.

| **T cell Proliferation** | | | | | |
|---|---|---|---|---|---|
| Plate 1 | Nab | | RU346 | RU350 | Media |
| A | 42 | 1 | 37.3 | 28 | 36361.9 |
| B | 40910.7 | | 94808.1 | 74.7 | 42663.8 |
| C | 52155.5 | | 55277.3 | 46179.1 | 48044.3 |
| D | 41607.6 | | 52138 | 54194.4 | 53253.6 |
| E | 36442 | | 49102.4 | 53277.2 | 46372.2 |
| F | 37988.7 | | 51285 | 58924.4 | 62391.8 |
| G | 37202.7 | | 53139.1 | 46056.3 | 52016.7 |
| H | 38818.1 | | 50602.8 | 52646.7 | 64420.5 |
| | | | | | |

| Plate 2 | Nab | | RU346 | RU350 | Media |
|---|---|---|---|---|---|
| A | 18.7 | | 14 | 42 | 68823.2 |
| B | 72188.2 | 1 | 108341.6 | 36745.1 | 51978.8 |
| C | 48534.9 | | 66042.8 | 62557.5 | 58244.3 |
| D | 65222 | | 49013.1 | 63867.2 | 62197.1 |
| E | 44970.2 | | 55537.9 | 66290 | 42555.2 |
| F | 36994.3 | | 59320.9 | 49054.3 | 58165.6 |
| G | 44920.7 | | 47373.7 | 60937.4 | 40598.4 |
| H | 34530 | | 52070.6 | 41897.1 | 42487 |
| | | | | | |

| Plate 3 | Nab | | RU346 | RU350 | Media |
|---|---|---|---|---|---|
| A | 46.7 | | 18.7 | 51.4 | 63111.1 |
| B | 78370.5 | | 84079.6 | 110594.7 | 51408.2 |
| C | 44235.7 | | 123241.3 | 58812.6 | 41777.8 |
| D | 55348.5 | | 61529 | 67792.8 | 79248.8 |
| E | 46765.1 | | 57697.5 | 70736.7 | 56283.9 |
| F | 35152.2 | | 72051.9 | 74224 | 68615.4 |
| G | 36359.1 | | 74186.6 | 59965.8 | 62897.8 |
| H | 39295.7 | | 72606.4 | 57220.4 | 42878.1 |

In the Table, "Nab" refers to Nabilone, "RU346" refers to Isomer D and "RU350" refers to Isomer B.

The results demonstrate that each of the compounds at the highest concentrations tested inhibit proliferation of T cells.

### EXAMPLE 8

### Pharmaceutical Compositions

### (i) Tablet Formulation - I

A tablet composition containing the dihydrotetrabenazine of the invention is prepared by mixing 50mg of the dihydrotetrabenazine with 197mg of lactose (BP) as diluent, and 3mg magnesium stearate as a lubricant and compressing to form a tablet in known manner.

### (ii) Tablet Formulation - II

A tablet composition containing the dihydrotetrabenazine of the invention is prepared by mixing the compound (25 mg) with iron oxide, lactose, magnesium stearate, starch maize white and talc, and compressing to form a tablet in known manner.

### (iii) Capsule Formulation

A capsule formulation is prepared by mixing 100mg of the dihydrotetrabenazine of the invention with 100mg lactose and filling the resulting mixture into standard opaque hard gelatin capsules.

### Equivalents

It will readily be apparent that numerous modifications and alterations may be made to the specific embodiments of the invention described above without departing from the principles underlying the invention.

## Claims

1. A 3,11b-*cis*-dihydrotetrabenazine compound of the formula (Ia): 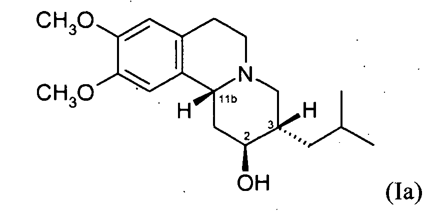 or a pharmaceutically acceptable salt thereof, for use in the prophylaxis or treatment of an inflammatory disease.

2. A 3,11b-*cis*-dihydrotetrabenazine compound for use according to claim 1 wherein the inflammatory disease is selected from rheumatoid arthritis, osteoarthritis, traumatic arthritis, gouty arthritis, rubella arthritis, psoriatic arthritis, and other arthritic conditions; acute or chronic inflammatory disease states such as the inflammatory reaction induced by endotoxin or inflammatory bowel disease; Reiter's syndrome, gout, rheumatoid spondylitis, chronic pulmonary inflammatory disease, Crohn's disease and ulcerative colitis.

3. A 3,11b-*cis*-dihydrotetrabenazine compound for use according to claim 2 wherein the inflammatory disease is rheumatoid arthritis.

4. A 3,11b-*cis*-dihydrotetrabenazine compound for use according to any one of claims 1 to 3 wherein the 3,11b-*cis*-dihydrotetrabenazine is in the form of an acid addition salt.

5. A 3,11b-*cis*-dihydrotetrabenazine compound for use according to claim 4 wherein the salt is a methane sulphonate salt.

6. The use of a 3,11b-*cis*-dihydrotetrabenazine compound of the formula (Ia): 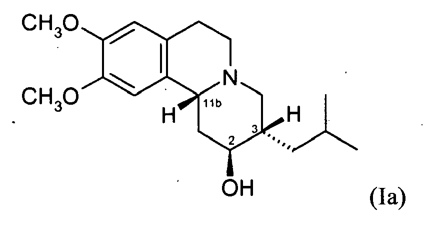 or a pharmaceutically acceptable salt thereof, for the manufacture of a medicament for the prophylaxis or treatment of an inflammatory disease.

7. The use according to claim 6 wherein the inflammatory disease is selected from rheumatoid arthritis, osteoarthritis, traumatic arthritis, gouty arthritis, rubella arthritis, psoriatic arthritis, and other arthritic conditions; acute or chronic inflammatory disease states such as the inflammatory reaction induced by endotoxin or inflammatory bowel disease; Reiter's syndrome, gout, rheumatoid spondylitis, chronic pulmonary inflammatory disease, Crohn's disease and ulcerative colitis.

8. The use according to claim 7 wherein the inflammatory disease is rheumatoid arthritis.

9. The use according to any one of claims 6 to 8 wherein the 3,11b-*cis-*dihydrotetrabenazine is in the form of an acid addition salt.

10. The use according to claim 9 wherein the salt is a methane sulphonate salt.

## Patentansprüche

1. 3,11b-*cis*-Dihydrotetrabenazinverbindung der Formel (Ia): 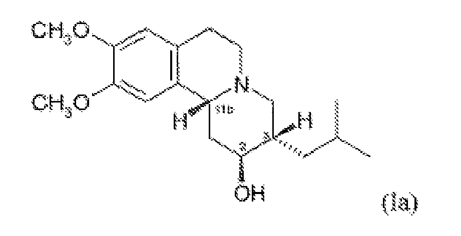 oder pharmazeutisch akzeptables Salz derselben zur Verwendung zur Prophylaxe oder Behandlung einer Entzündungskrankheit.

2. 3,11b-*cis*-Dihydrotetrabenazinverbindung zur Verwendung nach Anspruch 1, wobei die Entzündungskrankheit unter rheumatoider Arthritis, Osteoarthritis, posstraumatischer Arthrose, Gichtarthritis, Rötelnarthritis, Arthropatia psoriatica und anderen arthritischen Zuständen; akuten oder chronischen Entzündungskrankheitszuständen, wie der durch Endotoxin induzierten Entzündungsreaktion oder entzündlichen Darmerkrankung; Reiter-Krankheit, Gicht, Morbus Bechterew, chronischer Lungenentzündungskrankeit, Crohn-Krankheit und Colitis ulcerosa ausgewählt wird.

3. 3,11b-*cis*-Dihydrotetrabenazinverbindung zur Verwendung nach Anspruch 2, wobei die Entzündungskrankheit die rheumatoide Arthritis ist.

4. 3,11b-*cis*-Dihydrotetrabenazinverbindung zur Verwendung nach einem der Ansprüche 1 bis 3, wobei das 3,11b-*cis*-Dihydrotetrabenazin in Form eines sauren Additionssalzes vorliegt.

5. 3,11b-*cis*-Dihydrotetrabenazinverbindung zur Verwendung nach Anspruch 4, wobei das Salz ein Methansulfonatsalz ist.

6. Verwendung einer 3,11b-*cis*-Dihydrotetrabenazinverbindung der Formel (Ia): 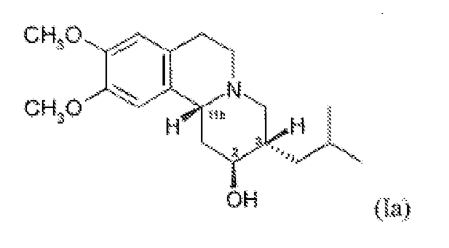 oder eines pharmazeutisch akzeptablen Salzes derselben zur Herstellung eines Medikaments zur Prophylaxe oder Behandlung einer Entzündungskrankheit.

7. Verwendung nach Anspruch 6, wobei die Entzündungskrankheit unter rheumatoider Arthritis, Osteoarthritis, posstraumatischer Arthrose, Gichtarthritis, Rötelnarthritis, Arthropatia psoriatica und anderen arthritischen Zuständen; akuten oder chronischen Entzündungskrankheitszuständen, wie der durch Endotoxin induzierten Entzündungsreaktion oder entzündlichen Darmerkrankung; Reiter-Krankheit, Gicht, Morbus Bechterew, chronischer Lungenentzündungskrankeit, Crohn-Krankheit und Colitis ulcerosa ausgewählt wird.

8. Verwendung nach Anspruch 7, wobei die Entzündungskrankheit die rheumatoide Arthritis ist.

9. Verwendung nach einem der Ansprüche 6 bis 8, wobei das 3,11b-*cis-*Dihydrotetrabenazin in Form eines sauren Additionssalzes vorliegt.

10. Verwendung nach Anspruch 9, wobei das Salz ein Methansulfonatsalz ist.

## Revendications

1. Composé 3,11b-*cis*-dihydrotétrabénazine ayant pour formule (Ia) : 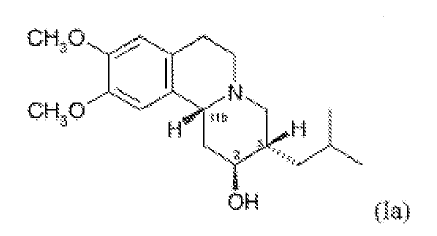 ou un sel dudit composé convenant à l'usage pharmaceutique, à utiliser dans la prophylaxie ou le traitement d'une maladie inflammatoire.

2. Composé 3,11b-*cis*-dihydrotétrabénazine à utiliser selon la revendication 1, dans laquelle la maladie inflammatoire est sélectionnée parmi le groupe constitué par la polyarthrite rhumatoïde, l'arthrose, l'arthrite traumatique, l'arthrite goutteuse, l'arthrite rubéoleuse, l'arthrite psoriasique et d'autres états arthritiques ; des états pathologiques inflammatoires aigus ou chroniques tels que la réaction inflammatoire induite par l'endotoxine ou les maladies inflammatoires chroniques de l'intestin ; le syndrome de Fiessinger-Leroy-Reiter, la goutte, la spondylite rhumatoïde, les maladies inflammatoires chroniques des poumons, la maladie de Crohn et la rectocolite hémorragique.

3. Composé 3,11b-*cis*-dihydrotétrabénazine à utiliser selon la revendication 2, dans laquelle la maladie inflammatoire est la polyarthrite rhumatoïde.

4. Composé 3,11b-*cis*-dihydrotétrabénazine à utiliser selon l'une quelconque des revendications 1 à 3, dans laquelle le composé 3,11b-*cis*-dihydrotétrabénazine se présente sous la forme d'un sel d'addition acide.

5. Composé 3,11b-*cis*-dihydrotétrabénazine à utiliser selon la revendication 4, dans lequel le sel est un sel méthane sulfonate.

6. Utilisation d'un composé 3,11b-*cis*-dihydrotétrabénazine ayant pour formule la formule (Ia) : 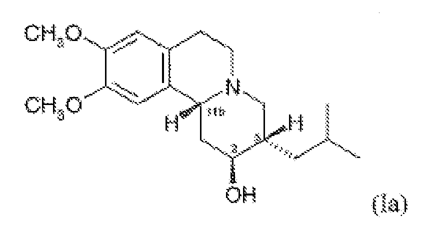 ou un sel dudit composé convenant à l'usage pharmaceutique, pour la fabrication d'un médicament destiné à la prophylaxie ou au traitement d'une maladie inflammatoire.

7. Utilisation selon la revendication 6, dans laquelle la maladie inflammatoire est sélectionnée parmi le groupe constitué par la polyarthrite rhumatoïde, l'arthrose, l'arthrite traumatique, l'arthrite goutteuse, l'arthrite rubéoleuse, l'arthrite psoriasique et d'autres états arthritiques ; des états pathologiques inflammatoires aigus ou chroniques tels que la réaction inflammatoire induite par l'endotoxine ou les maladies inflammatoires chroniques de l'intestin ; le syndrome de Fiessinger-Leroy-Reiter, la goutte, la spondylite rhumatoïde, les maladies inflammatoires chroniques des poumons, la maladie de Crohn et la rectocolite hémorragique.

8. Utilisation selon la revendication 7, dans laquelle la maladie inflammatoire est la polyarthrite rhumatoïde.

9. Utilisation selon l'une quelconque des revendications 6 à 8, dans laquelle 3,11b-*cis-*dihydrotétrabénazine se présente sous la forme d'un sel d'addition acide.

10. Utilisation selon la revendication 9, dans laquelle le sel est un sel méthane sulfonate.
